# EUROPEAN PATENT APPLICATION

(11) **EP 4 458 140 A1**
(43) Date of publication of application: **06.11.2024**
(21) Application number: 22915946.2
(22) Date of filing: 23.12.2022
(51) Int. Cl.: A01H 6/82, A01H 1/06, A01H 5/00, A24B 15/10, C12N 15/10, C12N 15/29

(54) **TOBACCO PLANT**

(30) Priority: 27.12.2021 JP 2021212001
(71) Applicant: Japan Tobacco Inc., Tokyo 105-6927 (JP)
(72) Inventor: SUZUKI, Shoichi, Tokyo 130-8603 (JP); UDAGAWA, Hisashi, Tokyo 130-8603 (JP); HIRASE, Taishi, Tokyo 130-8603 (JP)
(74) Representative: Hoffmann Eitle
(86) International application number: PCT/JP2022/047603
(87) International publication number: WO 2023/127723

(57) **Abstract**

The present invention relates to a tobacco plant, etc. This tobacco plant has either one or both of the following: (i) an endogenous gene containing, as an encoding region, a nucleic acid containing a base sequence encoding SEQ ID NO: 2 or an amino acid sequence having at least 95% identity with SEQ ID NO: 2, in which at least one codon corresponding to the amino acid sequence has mutated to a stop codon; and (ii) an endogenous gene containing, as an encoding region, a nucleic acid containing a base sequence encoding SEQ ID NO: 4 or an amino acid sequence having at least 95% identity with SEQ ID NO: 4, in which at least one codon corresponding to the amino acid sequence has mutated to a stop codon.

## Description

### TECHNICAL FIELD

The present invention relates a tobacco plant, a method for producing the tobacco plant, leaf tobacco harvested from the tobacco plant, and use thereof.

### BACKGROUND ART

Tobacco-specific nitrosamine (TSNA) is a nitrosated product of a tobacco alkaloid generated mainly at the time of curing tobacco leaves. Tobacco plants accumulate high levels of free nitrate in their leaves, and this relates to the generation of TSNA. Substances participating directly in TSNA generation at the time of curing tobacco leaves are recognized to be nitrite. Although the free nitrate accumulated in the leaves of tobacco plants is converted into nitrite by a nitrate reductase (NR) that tobacco has, nitrite has cytotoxicity and is promptly metabolized. Therefore, the amount of endogenous nitrite contained in plant tissue is generally very small. It is believed that most of the nitrite participating in TSNA generation at the time of curing tobacco leaves is produced from nitrate by microorganisms inhabiting phylloplanes. That is, as leaf tissue is decomposed in a process of curing tobacco leaves, nitrate accumulated in the leaves is eluted and converted into nitrite by a nitrate reductase (NR) that microorganisms inhabiting phylloplanes have. Once nitrite is formed, the compound binds to various tobacco alkaloids (such as pyridine-containing compound) to form nitrosamine.

Plant J., 2000, 21(3), pp. 259-267 describes that in a CLCa mutant of *Arabidopsis thaliana,* the nitrate amount in leaves decreases, and the nitrite amount increases. The nitrate amount accumulated in the leaves of *Arabidopsis thaliana* mutant was about 40% of that of a control. New Phytologist, 2009, 183, pp. 88-94 describes that in a CLCe mutant of *Arabidopsis thaliana,* the nitrate amount in leaves decreases, and the nitrite amount increases. It is assumed that in tobacco, as the nitrite amount increases, the amount of TSNA also increases.

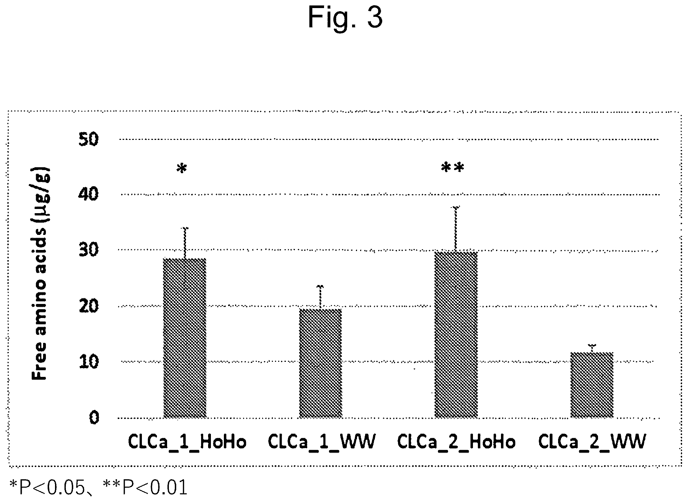

WO 2014/096283 describes that recombinants in which expressions of the CLC-NT2 gene (corresponding to an NtCLCa-S gene described later) and the NtCLCe gene that are assumed to be orthologs of tobacco corresponding to CLCa and CLCe of *Arabidopsis thaliana* have been suppressed by RNAi were produced. In every RNAi recombinants, gene expressions of CLC-NT2 and NtCLCe have been suppressed. In addition, in every RNAi recombinants, a decrease of nitrate in leaves was observed, but it is unclear whether the nitrate-decreasing effect is caused by CLC-NT2 or NtCLCe or by a synergistic effect. The degree of decrease in nitrate was about 40% to 50% (Example 6, Fig. 3).

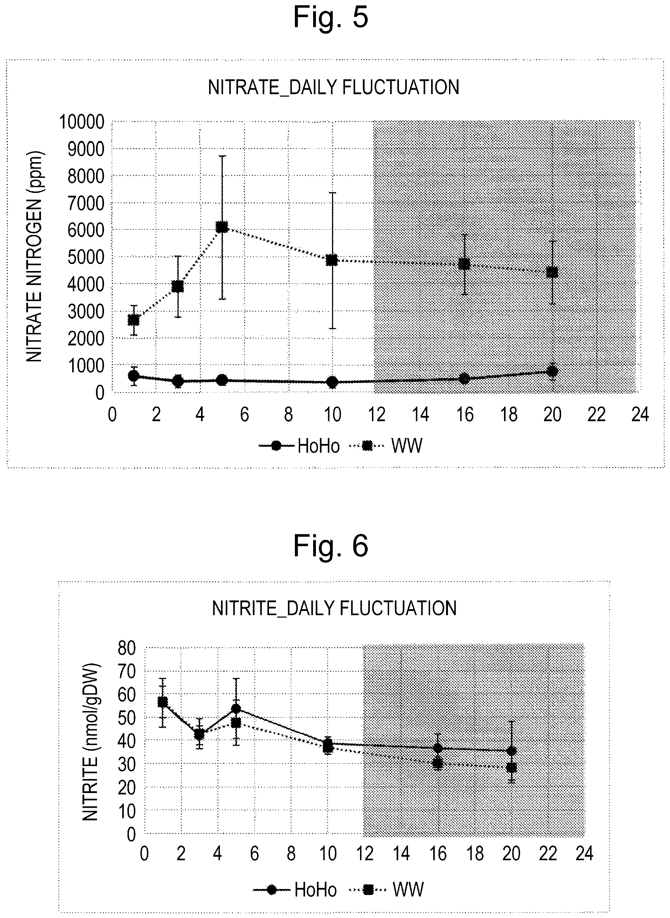

Mutant G163R of CLC-NT2 disclosed in WO 2014/096283 has characteristics that (i) while the nitrate amount in leaves from early morning to mid-morning is smaller than that in a control, it exceeds that in the control before noon (Example 8, Fig. 5) and that (ii) the degree of decrease in nitrate is also merely about 50% of that of the wild type. The nitrate amount in leaves of a P143L mutant of NtCLCe before noon was smaller than that of a control, but it exceeded that of the control in early morning (Example 9, Fig. 6). Both G163R mutant and P143L mutant are missense mutants.

### CITATION LIST

### PATENT LITERATURE

PTL 1: International Publication No. WO 2014/096283
PTL 2: International Publication No. WO 2022/124361

### NON PATENT LITERATURE

NPL 1: Plant J., 2000, 21(3), pp. 259-267
NPL 2: New Phytologist, 2009, 183, pp. 88-94
NPL 3: Nature, (2006), 442(7105), pp. 939-42
NPL 4: Communicative & Integrative Biology, 2010, 3(2), pp. 122-129
NPL 5: Mol. Genet. Genomics, (2010), 283, pp. 233-241
NPL 6: Phil. Trans. R. Soc. B, (2009), 364, pp. 195-201
NPL 7: BMC genomics, (2017), 18(1), pp. 1-14

### SUMMARY OF INVENTION

### TECHNICAL PROBLEM

It is known that nitrate contained in leaf tobacco participates in TSNA generation A method for decreasing largely (preferably 50% or more) the nitrate contained in leaf tobacco and development of a tobacco plant with decreased nitrate are being desired The present inventors have made extensive research efforts to solve the above problems and, as a result, have found that a tobacco plant having a stop codon in an NtCLCa-S gene and/or an NtCLCa-T gene has significantly decreased nitrate in the leaves without adversely affecting the growth compared to a control tobacco plant not having the stop codon and arrived at the present invention.

### SOLUTION TO PROBLEM

The present invention includes, but not limited to, the following embodiments.

### [Embodiment 1]

A tobacco plant including one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

### [Embodiment 2]

The tobacco plant according to embodiment 1, fulfilling one or both of the following requirements:
in the nucleic acid included in the endogenous gene of (i), at least one of codons corresponding to amino acids at positions from 233 to 285 of SEQ ID NO: 2 is mutated to a stop codon; and
in the nucleic acid included in the endogenous gene of (ii), at least one of codons corresponding to amino acids at positions from 233 to 285 of SEQ ID NO: 4 is mutated to a stop codon.

### [Embodiment 3]

The tobacco plant according to embodiment 1 or 2, including both endogenous genes of (i) and (ii).

### [Embodiment 4]

The tobacco plant according to any one of embodiment 1 to 3, wherein the endogenous gene of (i) and/or the endogenous gene of (ii) is a homozygote.

### [Embodiment 5]

The tobacco plant according to any one of embodiments 1 to 4, wherein in the nucleic acid included in the endogenous gene of (i), a codon corresponding to an amino acid at position 279 of SEQ ID NO: 2 is mutated to a stop codon.

### [Embodiment 6]

The tobacco plant according to any one of embodiments 1 to 5, wherein in the nucleic acid included in the endogenous gene of (ii), a codon corresponding to an amino acid at position 285 of SEQ ID NO: 4 is mutated to a stop codon.

### [Embodiment 7]

The tobacco plant according to any one of embodiments 1 to 5, wherein in the nucleic acid included in the endogenous gene of (ii), a codon corresponding to an amino acid at position 233 of SEQ ID NO: 4 is mutated to a stop codon.

### [Embodiment 8]

The tobacco plant according to any one of embodiments 1 to 6, wherein
in the nucleic acid included in the endogenous gene of (i), a codon corresponding to an amino acid at position 279 of SEQ ID NO: 2 is mutated to a stop codon, and
in the nucleic acid included in the endogenous gene of (ii), a codon corresponding to an amino acid at position 233 or position 285 of SEQ ID NO: 4 is mutated to a stop codon.

### [Embodiment 9]

The tobacco plant according to any one of embodiments 1 to 8, wherein in the nucleic acid included in the endogenous gene of (i), an amino acid corresponding to position 163 of SEQ ID NO: 2 encoded by the nucleic acid is glycine.

### [Embodiment 10]

The tobacco plant according to any one of embodiments 1 to 9, further including:
(iii) an endogenous gene including, as a coding region, a nucleic acid including a nucleotide sequence encoding an amino acid sequence of SEQ ID NO: 8 or having at least 95% identity with SEQ ID NO: 8 in which an amino acid corresponding to position 231 of the amino acid sequence is proline.

### [Embodiment 11]

The tobacco plant according to any one of embodiments 1 to 10, having one or more properties of the following (a) to (c):
(a) a nitrate content decreased compared to a control;
(b) a nitrite content equivalent to a control; and
(c) an amino acid content increased compared to a control,
wherein the control is a tobacco plant including a polypeptide containing the amino acid sequence of SEQ ID NO: 2 and a polypeptide containing the amino acid sequence of SEQ ID NO: 4.

### [Embodiment 12]

The tobacco plant according to embodiment 11, wherein nitrate is decreased by at least 80% compared to the control.

### [Embodiment 13]

The tobacco plant according to any one of embodiments 1 to 12, wherein the tobacco plant is a mutant or a gene-modified variant.

### [Embodiment 14]

The tobacco plant according to any one of embodiments 1 to 13, wherein the tobacco plant is *Nicotiana tabacum.*

### [Embodiment 15]

A tobacco plant including one or both of:
an endogenous gene including, as a coding region, a nucleotide sequence encoding a polypeptide lacking an amino acid sequence from an amino acid residue between positions 233 to 285 to a C-terminal amino acid residue in an amino acid sequence of SEQ ID NO: 2 or having at least 95% identity with SEQ ID NO: 2; and
an endogenous gene including, as a coding region, a nucleotide sequence encoding a polypeptide lacking an amino acid sequence from an amino acid residue between positions 233 to 285 to a C-terminal amino acid residue in an amino acid sequence of SEQ ID NO: 4 or having at least 95% identity with SEQ ID NO: 4.

### [Embodiment 16]

A method for producing the tobacco plant according to any one of embodiments 1 to 14, including:
(i) introducing a mutation into a tobacco plant such that in an endogenous gene including, as a coding region, a nucleic acid including a nucleotide sequence encoding an amino acid sequence of SEQ ID NO: 2 or having at least 95% identity with SEQ ID NO: 2, at least one of codons corresponding to the amino acid sequence is mutated to a stop codon; and/or
(ii) introducing a mutation into the tobacco plant such that in an endogenous gene including, as a coding region, a nucleic acid including a nucleotide sequence encoding an amino acid sequence of SEQ ID NO: 4 or having at least 95% identity with SEQ ID NO: 4, at least one of codons corresponding to the amino acid sequence is mutated to a stop codon.

### [Embodiment 17]

A method for producing the tobacco plant according to any one of embodiments 1 to 14, including selecting a tobacco plant including one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

### [Embodiment 18]

The method according to embodiment 16 or 17, further including:
selecting a tobacco plant having a decreased nitrate content.

### [Embodiment 19]

Leaf tobacco harvested from the tobacco plant according to any one of embodiments 1 to 14.

### [Embodiment 20]

A cured leaf generated from the leaf tobacco according to embodiment 19.

### [Embodiment 21]

A cut filler, powder, sheet, stem, granule, or extract produced from the cured leaf according to embodiment 20.

### [Embodiment 22]

A tobacco product containing the cured leaf according to embodiment 20 and/or the cut filler, powder, sheet, stem, granule, or extract according to embodiment 21.

### ADVANTAGEOUS EFFECTS OF INVENTION

In the tobacco plant of the present invention including a stop codon in an NtCLCa-S gene and/or an NtCLCa-T gene, nitrate in leaves decreases significantly (preferably about 80% or more) compared to a control tobacco plant not including the stop codon in these genes. TSNA in a tobacco raw material or tobacco product can be decreased by using the tobacco plant of the present invention. In the tobacco plant of the present invention, not only the nitrate amount in leaves decreases, but also the nitrite amount in leaves does not increase, and preferably, the growth of the plant body is also good, and no increase in the adaptation cost in cultivation, such as delayed flowering, is observed In the tobacco plant of the present invention, the amino acid content in leaves also increases. It is known that amino acids affect the flavor and taste of tobacco. Accordingly, the increase in amino acid content improves the flavor and taste and is an excellent noticeable effect also for using the tobacco as a material of leaf tobacco for producing a tobacco product.

### BRIEF DESCRIPTION OF DRAWINGS

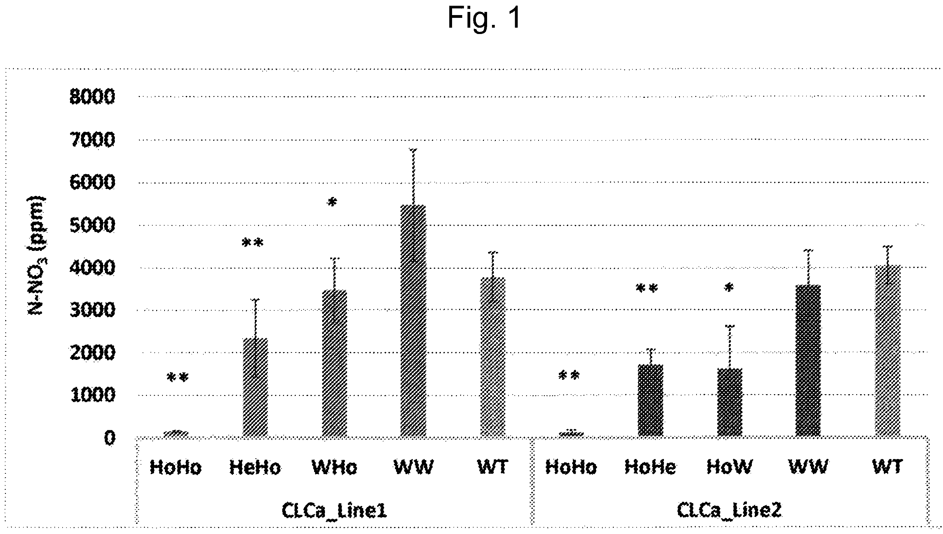

[Fig. 1] Fig. 1 shows the nitrate amount in leaves (laminas) of each of mutants and wild type. Ho, He, and W indicate that the NtCLCa gene is a homozygote of a mutant allele, is a heterozygote of a mutant allele and a wild type allele, and is a wild type without a mutation, respectively. A genotype of an NtCLCa-S gene and a genotype of an NtCLCa-T gene are shown in this order, and, for example, HeHo indicates that the NtCLCa-S gene is a heterozygote of a mutant allele and a wild type allele and the NtCLCa-T gene is a homozygote of a mutant allele. WT is a homozygote of a wild type gene and is a tobacco (variety: Tsukuba 1) as a control. The numbers of replications are 4 in WT and 5 in all others, and the error bars indicate standard deviations. Significant difference by a t-test: * P < 0.05, ** P < 0.01.

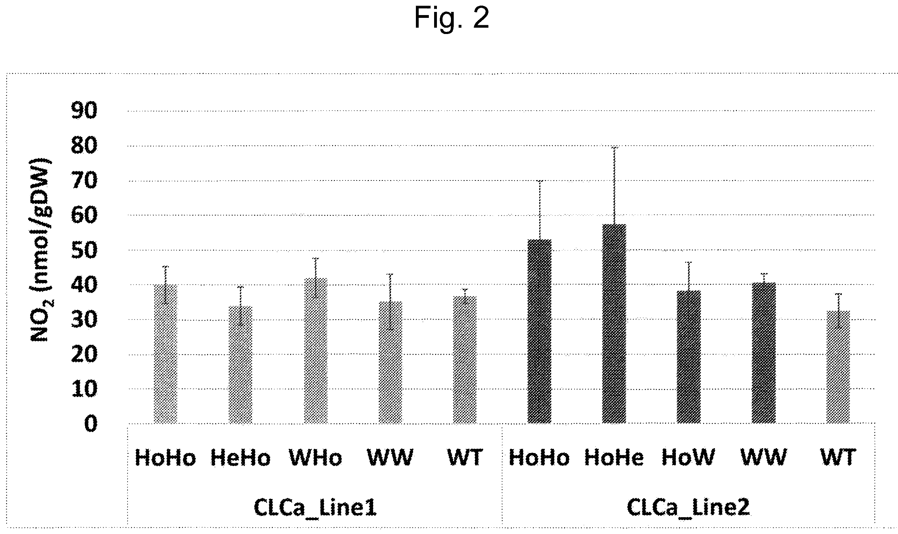

[Fig. 2] Fig. 2 shows the nitrite amount in leaves (laminas) of each of mutants and wild type. Ho, He, and W indicate that the NtCLCa gene is a homozygote of a mutant allele, is a heterozygote of a mutant allele and a wild type allele, and is a wild type without a mutation, respectively. A genotype of an NtCLCa-S gene and a genotype of an NtCLCa-T gene are shown in this order, and, for example, HeHo indicates that the NtCLCa-S gene is a heterozygote of a mutant allele and a wild type allele and the NtCLCa-T gene is a homozygote of a mutant allele. WT is a wild type tobacco (variety: Tsukuba 1). The numbers of replications are 4 in WT and 5 in all others, and the error bars indicate standard deviations.
[Fig. 3] Fig. 3 shows the free amino acid amount in leaves (laminas) of each of mutants and wild type. Ho and W indicate that the NtCLCa gene is a homozygote of a mutant allele and is a wild type without a mutation, respectively. A genotype of an NtCLCa-S gene and a genotype of an NtCLCa-T gene are shown in this order, and, for example, HeHo indicates that the NtCLCa-S gene is a heterozygote of a mutant allele and a wild type allele and the NtCLCa-T gene is a homozygote of a mutant allele. The number of replication is 5, and the error bars indicate standard deviations. Significant difference by a t-test: * P < 0.05, ** P < 0.01.

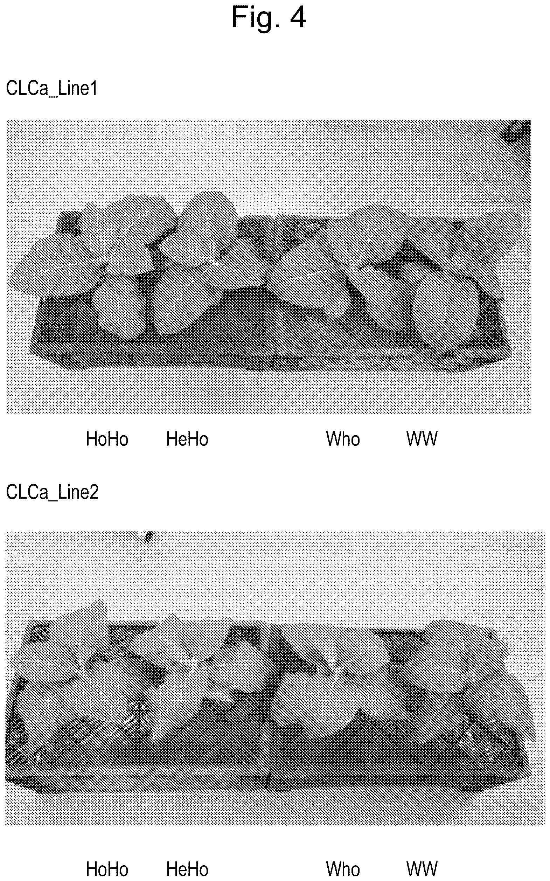

[Fig. 4] Fig. 4 is photographs showing the growth of each of mutants and wild type tobacco plants at the time of sampling (20 days after transplantation). Ho, He, and W indicate that the NtCLCa gene is a homozygote of a mutant allele, is a heterozygote of a mutant allele and a wild type allele, and is a wild type without a mutation, respectively. A genotype of an NtCLCa-S gene and a genotype of an NtCLCa-T gene are shown in this order, and, for example, HeHo indicates that the NtCLCa-S gene is a heterozygote of a mutant allele and a wild type allele and the NtCLCa-T gene is a homozygote of a mutant allele.
[Fig. 5] Fig. 5 is a graph showing daily fluctuations of the nitrate amount in the leaves (laminas) of HoHo and WW. Ho and W indicate that the NtCLCa gene is a homozygote of a mutant allele and is a wild type without a mutation, respectively. A genotype of an NtCLCa-S gene and a genotype of an NtCLCa-T gene are shown in this order, and, for example, HoHo indicates that the NtCLCa-S gene and the NtCLCa-T gene are both homozygotes of mutant alleles. The numbers of replications are 3 in HoHo in a dark place for 8 hours and 4 in all others, and the error bars indicate standard deviations. In Fig. 5, black circles show the results of HoHo and black squares show the results of WW.
[Fig. 6] Fig. 6 is a graph showing daily fluctuations of the nitrite amount in the leaves (laminas) of HoHo and WW. Ho and W indicate that the NtCLCa gene is a homozygote of a mutant allele and is a wild type without a mutation, respectively. A genotype of an NtCLCa-S gene and a genotype of an NtCLCa-T gene are shown in this order, and, for example, HoHo indicates that the NtCLCa-S gene and the NtCLCa-T gene are both homozygotes of mutant alleles. The numbers of replications are 3 in HoHo in a dark place for 8 hours and 4 in all others, and the error bars indicate standard deviations. In Fig. 6, black circles show the results of HoHo and black squares show the results of WW.

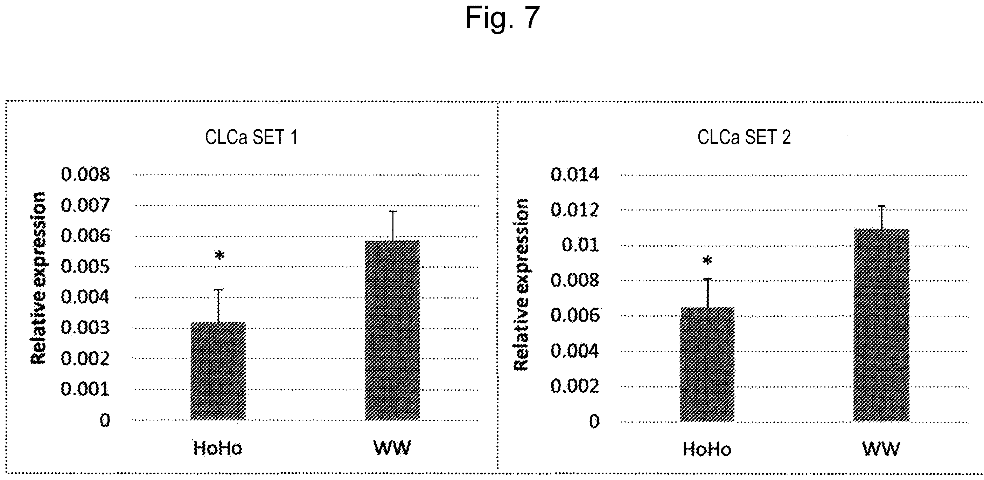

[Fig. 7] Fig. 7 shows the results of analysis of NtCLCa gene expression levels in an NtCLCa mutant. The results are shown as the average values of relative quantification (Δ Δ Ct method) of the target expression levels using ribosomal protein L25 (Accession No. L18908) as a control gene. The number of replication is 10, and the error bars indicate standard deviations. Significant difference by a t-test: *P < 0.05. The expression level of the NtCLCa gene in HoHo was significantly low, about 1/2, compared to that in WW in both 2 sets of primers used.

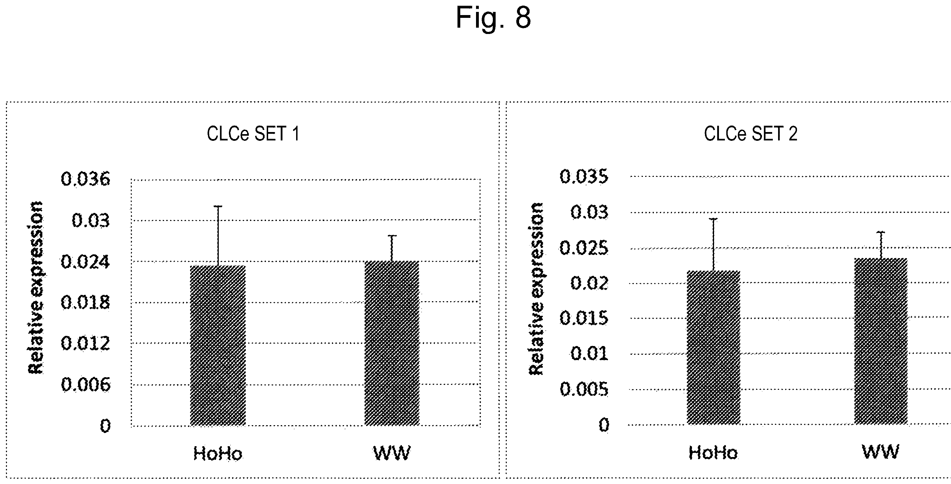

[Fig. 8] Fig. 8 shows the results of analysis of NtCLCe gene expression levels in the NtCLCa mutant. The results are shown by the average values of relative quantification (Δ Δ Ct method) of the target expression levels using ribosomal protein L25 (Accession No. L18908) as a control gene. There was no significant difference between HoHo and WW in the expression level of the NtCLCe gene. The number of replication is 10, and the error bars indicate standard deviations.

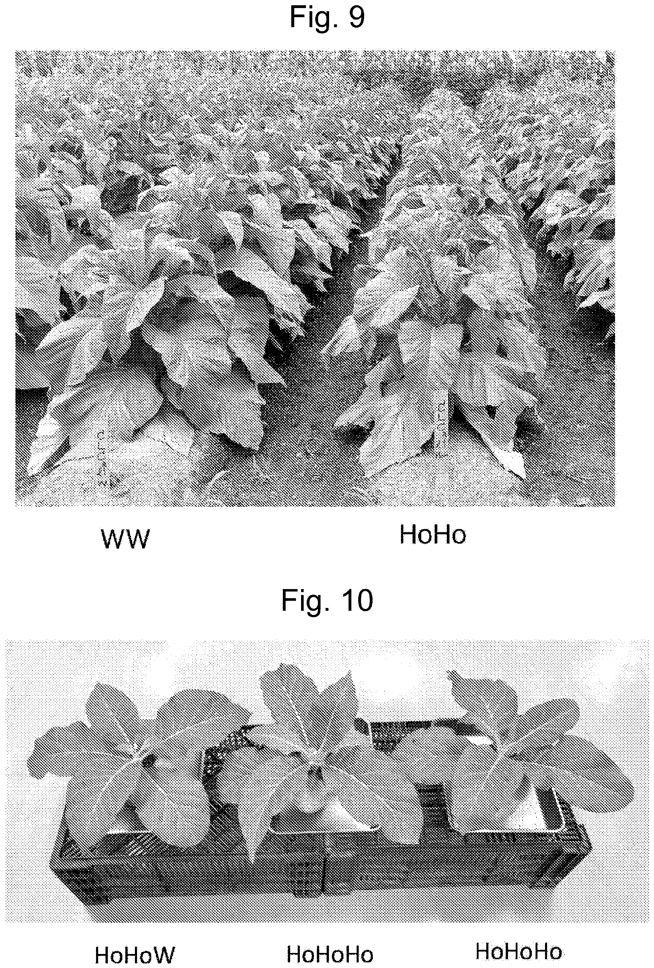

[Fig. 9] Fig. 9 is photographs showing the growth of each of mutants and wild type tobacco plants 54 days after transplantation. Ho and W indicate that the NtCLCa gene is a homozygote of a mutant allele and is a wild type without a mutation, respectively. A genotype of an NtCLCa-S gene and a genotype of an NtCLCa-T gene are shown in this order, and, for example, HoHo indicates that the NtCLCa-S gene and the NtCLCa-T gene are both homozygotes of mutant alleles.
[Fig. 10] Fig. 10 is photographs showing the growth of each of mutants and wild type tobacco plants 20 days after transplantation (the time of sampling). Ho and W indicate that the NtCLCa gene and NtNIA1 gene are homozygotes of mutant alleles and are wild types without a mutation, respectively. A genotype of an NtCLCa-S gene and genotypes of an NtCLCa-T gene and an NtNIA1 gene are shown in this order. In HoHoHe, both the NtCLCa-S gene and the NtCLCa-T gene are homozygote of mutant alleles, and the NtNIA1 gene is a heterozygote of a mutant allele and a wild type allele.

### DESCRIPTION OF EMBODIMENTS

The present invention includes the following embodiments unlimitedly Technical and scientific terms used herein have the same meanings as those that a person skilled in the art usually understand unless otherwise specified. Substances, materials, and examples disclosed herein are mere illustrations, and are not intended to limit the present invention. When things "in one embodiment" is mentioned, it is meant that the things are not limited to the embodiment, namely that the things are unlimited.

### 1. Tobacco plant

In one embodiment, the present invention relates to a tobacco plant. The tobacco plant of the present invention includes one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

The term "endogenous gene" means a gene that is not introduced into a target tobacco plant from outside the body, but is instead a gene that is present endogenously in the genome of the tobacco plant. The "endogenous gene" used in the present invention is not a wild type gene including a nucleic acid encoding an amino acid sequence of a so-called wild type protein (e.g., SEQ ID NO: 2 and SEQ ID NO: 4), but instead a gene including a mutation as described above. The endogenous gene of (i) and/or the endogenous gene of (ii) may be a heterozygote of a mutant gene including the above-mentioned mutation and a gene not including the above-mentioned mutation or a homozygote of a mutant gene including the above-mentioned mutation.

The "tobacco plant" is a plant of *Solanaceae Nicotina,* and examples thereof include *Nicotiana acaulis, Nicotiana acuminata, Nicotiana acuminata var. multzjlora, Nicotiana africana, Nicotiana alata, Nicotiana amplexicaulis, Nicotiana arentsii, Nicotiana attenuata, Nicotiana benavidesii, Nicotiana benthamiana, Nicotiana bigelovii, Nicotiana bonariensis, Nicotiana cavicola, Nicotiana clevelandii, Nicotiana cordifolia, Nicotiana corymbosa, Nicotiana debneyi, Nicotiana excelsior, Nicotiana forgetiana, Nicotiana fragrans, Nicotiana glauca, Nicotiana glutinosa, Nicotiana goodspeedii, Nicotiana gossei, Nicotiana ingulba, Nicotiana kawakamii, Nicotiana knightiana, Nicotiana langsdorfi, Nicotiana linearis, Nicotiana longiflora, Nicotiana maritima, Nicotiana megalosiphon, Nicotiana miersii, Nicotiana noctiflora, Nicotiana nudicaulis, Nicotiana obtusifolia, Nicotiana occidentalis, Nicotiana occidentalis subsp. Hesperis, Nicotiana otophora, Nicotiana paniculata, Nicotiana pauczjlora, Nicotiana petunioides, Nicotiana plumbaginifolia, Nicotiana quadrivalvis, Nicotiana raimondii, Nicotiana repanda, Nicotiana rosulata, Nicotiana rosulata subsp. Ingulba, Nicotiana rotundifolia, Nicotiana rustica* (Wild tobacco), *Nicotiana setchellii, Nicotiana simulans, Nicotiana solanifolia, Nicotiana spegauinii, Nicotiana stocktonii, Nicotiana suaveolens, Nicotiana sylvestris, Nicotiana tabacum, Nicotiana thyrsiflora, Nicotiana tomentosa, Nicotiana tomentosifomis, Nicotiana trigonophylla, Nicotiana umbratica, Nicotiana undulata, Nicotiana velutina, Nicotiana wigandioides,* and hybrids of *Nicotiana* plants. The tobacco plant is, but not limited to, more preferably *Nicotiana benthamiana, Nicotiana rustica,* or *Nicotiana tabacum,* and *Nicotiana rustic a* and *Nicotiana tabacum* that are used as raw materials for leaf tobacco production are particularly preferable.

In one embodiment, the tobacco plant is *Nicotiana tabacum.*

The tobacco plant can include not only a mature tobacco plant and the whole thereof but also portions thereof. The portions are unlimitedly selected from the group consisting of leaves (including leaf blades and petioles), stems, roots, seeds, flowers, pollens, anthers, ovules, pedicels, meristematic tissues, cotyledons, hypocotyls, pericycles, embryos, albumens, explants, calluses, tissue cultures, sprouts, cells, and protoplast.

The tobacco plant includes one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

*Nicotiana tabacum* is an amphidiploid plant, and the genome thereof includes a genome (S genome) derived from an ancestor species of *Nicotiana sylvestris* and a genome (T genome) derived from an ancestor species of *Nicotiana tomentosiformis.* In many cases, as the genes of *Nicotiana tabacum,* there are two types of genes: a gene derived from the S genome and a gene derived from the T genome.

SEQ ID NO: 2 is a tobacco homolog of AtCLCa which is a chloride channel (CLC) family of *Arabidopsis,* and is the amino acid sequence of NtCLCa-S protein encoded by an NtCLCa-S gene located on the S genome of *Nicotiana tabacum* (Nt). SEQ ID NO: 1 is the nucleotide sequence of a coding region (CDS) of the NtCLCa-S gene. SEQ ID NO: 4 is a tobacco homolog of AtCLCa, and is the amino acid sequence of NtCLCa-T protein encoded by an NtCLCa-T gene located on the T genome of *Nicotiana tabacum* (Nt). SEQ ID NO: 3 is the nucleotide sequence of CDS of the NtCLCa-T gene. The homology between the amino acid sequence of the NtCLCa-S protein and the amino acid sequence of the NtCLCa-T protein is 98%, and the homology between the CDS of the NtCLCa-S gene and the CDS of the NtCLCa-T gene is 97%.

The CLC family is a group of proteins constituting a group of potential-dependent ion channels. In a plant body, a chloride channel is involved in transfer of various anions (Phil. Trans. R. Soc. B, (2009), 364, 195-201), and contributes to a large number of plant-specific functions such as regulation of turgor pressure, stomal movement, nutrient transport, and/or metal tolerance and similar things.

*Arabidopsis* CLCa (AtCLCa) mediates accumulation of nitrate in the plant vacuole as a nitrate/proton antiporter (Nature (2006), 442 (7105): 939-42). This literature describes that as a result of analysis by an electrophysiological analysis method, AtCLCa is a 2NO³⁻/1H⁺ exchanger that can specifically accumulate nitrate ions in the vacuole. In order to test the nitrate-transporting activity ofNtCLCa, a similar method can be used. Communicative & Integrative Biology, 2010, 3(2), 122-129 and New Phytol., 2009, 183(1): 88-94 disclose that AtCLCe may participate in transportation of nitrite incorporated by the nitrite transporter of the chloroplast envelope from the stroma of chloroplasts to the thylakoid. Unlimitedly, the method for measuring this activity described in Nature, (2006), 442 (7105): 939-42 can be used in the measurement of the activity of NtCLCe.

It is assumed that NtCLCa herein is an ortholog of *Arabidopsis* CLCa (AtCLCa) from the viewpoint of the sequence homology and function.

The nucleotide sequence included in the nucleic acid may be a nucleotide sequence encoding an amino acid sequence of SEQ ID NO: 2 or having at least 95% identity with SEQ ID NO: 2. Alternatively, the nucleotide sequence may be a nucleotide sequence encoding an amino acid sequence having at least 95% identity with SEQ ID NO: 4. In one embodiment, the identity is at least 96%, at least 97%, at least 98%, or at least 99%.

Herein, the identity % between two amino acid sequences can be determined by visual inspection and mathematical calculation. The identity % can also be determined using a computer program Examples of the computer program include BLAST and ClustalW. In particular, various conditions (parameters) for identity retrieval by the BLAST program are described by Altschul, et al. (Nucl. Acids Res., 25, pp. 3389-3402, 1997) and can be publicly obtained from the web sites of the NCBI and the DNA Data Bank of Japan (DDBJ) (BLAST manual, Altschul, et al., NCB/NLM/NIH Bethesda, MD20894; Altschul, et al.). The identity % can also be determined using a program such as genetic information processing software GENETYX (GENETYX Corporation), DNASIS Pro (Hitachi Solutions, Ltd.), or Vector NTI (Infomax).

Herein, the identity % of two nucleotide sequences can be determined by visual inspection and mathematical calculation. The identity % can also be determined using a computer program Examples of the sequence comparison computer program include the BLASTN program (Altschul, et al., (1990), J. Mol. Biol., 215: pp.403-10): version 2.2.7 that is available from the web site of the United States National Library of Medicine: https://blast.ncbi.nlm.nih.gov/Blast.cgi and the WU-BLAST 2.0 algorithm. The standard default parameters of WU-BLAST 2.0 can be set as described in the following Internet site: http://blast.wustl.edu.

"An amino acid sequence having at least 95% identity with SEQ ID NO: 2 or an amino acid sequence having at least 95% identity with SEQ ID NO: 4" may be an amino acid sequence in which one or several amino acids are deleted, substituted, inserted, or added in the amino acid sequence of SEQ ID NO: 2 or 4. The term "one or several amino acids are deleted, substituted, inserted, or added" refers to an amino acid sequence in which one or several amino acids are deleted from the target amino acid sequence, one or several amino acids in the target amino acid sequence are substituted with other amino acids, other amino acids are inserted in the target amino acid sequence, and/or other amino acids are added to the target amino acid sequence. The "several amino acids" unlimitedly means 40 or less, 35 or less, 30 or less, 25 or less, 20 or less, 15 or less, 12 or less, 10 or less, 8 or less, 6 or less, 5 or less, 4 or less, 3 or less, or 2 or less amino acids. Alternatively, the several amino acids means amino acids corresponding to 5%, preferably 4%, 3%, 2%, or 1% of the full-length of the amino acid sequence

The substitution of amino acid residues in an amino acid sequence having at least 95% identity with SEQ ID NO: 2 or an amino acid sequence having at least 95% identity with SEQ ID NO: 4 is preferably conservative substitution. The conservative substitution is to substitute a specific amino acid residue with a residue having similar physicochemical characteristics and may be any substitution as long as the characteristics of the original sequence structure are not substantially changed. For example, as long as the substituted amino acid does not destroy a helix present in the original sequence or does not destroy the secondary structure of another type characterizing the original sequence, any substitution may be performed. Examples of the conservative substitution of amino acid residues are categorized below by each substitutable residue, but the substitutable amino acid residues are not limited to those described below.
Group A: leucine, isoleucine, valine, alanine, methionine, glycine, cysteine, and proline;
Group B: aspartic acid and glutamic acid;
Group C: asparagine and glutamine;
Group D: lysine and arginine;
Group E: serine and threonine; and
Group F: phenylalanine, tyrosine, tryptophan, and histidine.

In nonconservative substitution, among the above-mentioned types, one member can be replaced with a member of another type. For example, in order to eliminate inadvertent sugar chain modification, an amino acid in the above-mentioned groups B, D, and E may be substituted with an amino acid in other groups Alternatively, in order to prevent folding into a tertiary structure in a protein, cysteine may be deleted or substituted with another amino acid. Alternatively, in order to maintain the balance between hydrophobicity and hydrophilicity or to increase the degree of hydrophilicity for facilitating synthesis, an amino acid may be substituted in view of the amino acid hydropathy index (J. Kyte and R. Doolittle, J. Mol Biol., Vol. 157, pp. 105-132, 1982), which is an indicator of hydrophobicity/hydrophilicity of an amino acid.

As another embodiment, substitution with an amino acid having less steric hindrance than the original amino acid, for example, substitution of an amino acid in group F with an amino acid in group A, B, C, D, or E may be performed; or substitution of a charged amino acid with an uncharged amino acid, for example, substitution of an amino acid in group B with an amino acid in group C may be performed

Proteins including or consisting of an amino acid sequence having at least 95% identity with SEQ ID NO: 2 or an amino acid sequence having at least 95% identity with SEQ ID NO: 4 have a function that is possessed by proteins including or consisting of the amino acid sequence of SEQ ID NO: 2 or the amino acid sequence of SEQ ID NO: 4, respectively, and preferably have the function of CLCa of the CLC family. Having the function of CLCa means nitrate ions can be specifically accumulated in the vacuole of the plant body and to function as a 2NO³⁻/1H⁺ exchanger.

In "a nucleotide sequence encoding an amino acid sequence of SEQ ID NO: 2 or having at least 95% identity with SEQ ID NO: 2" or "a nucleotide sequence encoding an amino acid sequence of SEQ ID NO: 4 or having at least 95% identity with SEQ ID NO: 4", at least one of codons corresponding to the amino acid sequence is mutated to a stop codon. By mutating at least one of codons corresponding to the amino acid sequence to a stop codon, a protein lacking the amino acid residues after (downstream) the former amino acid residue corresponding to the stop codon can be produced. Alternatively, the production of the protein may not occur.

The tobacco plant includes one or both of (i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon, and (ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon. In one embodiment, both the endogenous genes are included

In one embodiment, the tobacco plant fulfills one or both of the following requirements:
in the nucleic acid included in the endogenous gene of (i), at least one of codons corresponding to amino acids at positions from 233 to 285 of SEQ ID NO: 2 is mutated to a stop codon; and
in the nucleic acid included in the endogenous gene of (ii), at least one of codons corresponding to amino acids at positions from 233 to 285 of SEQ ID NO: 4 is mutated to a stop codon.
In one embodiment, both requirements are fulfilled.

The "corresponding to the amino acids at positions from 233 to 285 of SEQ ID NO: 2" means amino acid residues that can be understood to "correspond to amino acids at positions from 233 to 285 of SEQ ID NO: 2" from the information on peripheral amino acid sequences when "the amino acid sequence having at least 95% identity with SEQ ID NO: 2" is compared to SEQ ID NO: 2, and the amino acid residues do not necessarily have to perfectly match the amino acid positions from 233 to 285. For example, when an amino acid sequence that is not present in SEQ ID NO: 2 is added to the N-terminal or even when an amino acid residue in the middle is deleted, amino acid residues that can be understood to "correspond to amino acids at positions from 233 to 285 of SEQ ID NO: 2" can be recognized by comparing with SEQ ID NO: 2. "Corresponding to the amino acids at positions from 233 to 285 of SEQ ID NO: 4" is synonymous with the above.

In Examples, two mutants of a polypeptide translated from the NtCLCa-T gene: a mutant (222T) in which the codon encoding the 233rd tryptophan (W) was mutated to a stop codon and a mutant (223T) in which the codon encoding the 285th tryptophan (W) was mutated to a stop codon, were obtained, and it was confirmed that both the mutants had an effect of decreasing nitrate. Accordingly, a tobacco plant including at least an endogenous gene including, as a coding region, a nucleic acid included in the endogenous gene of (ii) in which at least one of codons corresponding to the amino acids at positions from 233 to 285 of SEQ ID NO: 4 is mutated to a stop codon can obtain a similar effect.

In addition, a mutant (220S) in which the codon encoding the 279th tryptophan (W) in a polypeptide translated from the NtCLCa-S gene was mutated to a stop codon was obtained, and it was confirmed that this mutant had an effect of decreasing nitrate. The amino acid sequences at positions from 233 to 285 of SEQ ID NO: 2 and SEQ ID NO: 4 are the same. Accordingly, as in the NtCLCa-T gene, also in the NtCLCa-S gene, it is inferred that a tobacco plant including at least an endogenous gene including, as a coding region, a nucleic acid included in the endogenous gene of (i) in which at least one of codons corresponding to the amino acids at positions from 233 to 285 of SEQ ID NO: 2 is mutated to a stop codon can obtain a similar effect. This is consistent with the results that suggest NtCLCa-S and NtCLCa-T described in Examples have equivalent nitrate transport capacities.

The mutation to a "stop codon" in the present invention includes a change of a corresponding codon site (only) to a stop codon by a nonsense mutation occurred in the NtCLCa gene and also a mutation to a "stop codon" as a result of a frameshift caused by insertion or deletion of a nucleotide in the NtCLCa gene. In the latter, the insertion or deletion of a nucleotide after the 697th position of the CDS sequence of SEQ ID NO: 1 or 3 is preferably performed

In one embodiment, in a nucleic acid included in the endogenous gene of (i), at least one of codons corresponding to the amino acids at positions 233, 235, 238, 246, 251, 253, 254, 255, 258, 273, 278, 279, 280, and 285 of SEQ ID NO: 2 is mutated to a stop codon by a nonsense mutation due to single nucleotide substitution.

In one embodiment, in a nucleic acid included in the endogenous gene of (i), at least one of codons corresponding to the amino acids at positions 233, 235, 238, 240, 241, 242, 244, 245, 247, 251, 258, 278, 279, 280, 281, and 285 from the beginning of the amino acid sequence that can be translated is mutated to a stop codon by a frameshift due to insertion of one nucleotide.

In one embodiment, in a nucleic acid included in the endogenous gene of (i), the nucleotides from the 794th to 796th are changed to TAG and the nucleotides from the 815th to 817th are changed to TAG in the CDS sequence of SEQ ID NO: 1 by deletion of one nucleotide, and thereby the codons corresponding to the amino acids at positions 265 and 272 of SEQ ID NO: 2 are mutated to stop codons.

In one embodiment, in a nucleic acid included in the endogenous gene of (ii), at least one of codons corresponding to the amino acids at positions 233, 235, 238, 246, 251, 253, 254, 255, 258, 273, 278, 279, 280, and 285 of SEQ ID NO: 4 is mutated to a stop codon by a nonsense mutation due to single nucleotide substitution.

In one embodiment, in a nucleic acid included in the endogenous gene of (ii), at least one of codons corresponding to the amino acids at positions 233, 235, 238, 240, 241, 242, 244, 245, 247, 251, 258, 278, 279, 280, 281, and 285 from the beginning of the amino acid sequence that can be translated is mutated to a stop codon by a frameshift due to insertion of one nucleotide.

In one embodiment, in a nucleic acid included in the endogenous gene of (ii), the nucleotides from the 794th to 796th are changed to TAG and the nucleotides from the 815th to 817th are changed to TAG in the CDS sequence of SEQ ID NO: 3 by deletion of one nucleotide, and thereby the codons corresponding to the amino acids at positions 265 and 272 of SEQ ID NO: 4 are mutated to stop codons.

In one embodiment, in the nucleic acid included in the endogenous gene of (i), a codon corresponding to the amino acid at position 279 of SEQ ID NO: 2 is mutated to a stop codon.

In one embodiment, in the nucleic acid included in the endogenous gene of (ii), a codon corresponding to the amino acid at position 285 of SEQ ID NO: 4 is mutated to a stop codon.

In one embodiment, in the nucleic acid included in the endogenous gene of (ii), a codon corresponding to the amino acid at position 233 of SEQ ID NO: 4 is mutated to a stop codon.

In one embodiment,
in the nucleic acid included in the endogenous gene of (i), a codon corresponding to the amino acid at position 279 of SEQ ID NO: 2 is mutated to a stop codon; and
in the nucleic acid included in the endogenous gene of (ii), a codon corresponding to the amino acid at position 233 or position 285 of SEQ ID NO: 4 is mutated to a stop codon.

The endogenous gene of (i) and/or the endogenous gene of (ii) may be a heterozygote of a mutant gene including the above-mentioned mutation and a gene not including the above-mentioned mutation or may be a homozygote of a mutant gene including the above-mentioned mutation. Here, the homozygote includes not only a combination of alleles in which the positions of mutation to a stop codon are the same but also a combination of alleles in which the positions of mutation to a stop codon are different, as long as both alleles are mutant genes including the above-mentioned mutations. The endogenous gene of (i) and/or the endogenous gene of (ii) is preferably a homozygote of a mutant gene including the above-mentioned mutation. Preferably, the endogenous gene of (i) and the endogenous gene of (ii) are included, and both of them are homozygotes of mutant genes including the above-mentioned mutations, i.e., an individual whose genotype of NtCLCa is sstt. Here, S means an NtCLCa-S gene not including the above-mentioned mutation, s means an NtCLCa-S gene including the above-mentioned mutation, T means an NtCLCa-T gene not including the above-mentioned mutation, and t means an NtCLCa-T gene including the above-mentioned mutation.

In one embodiment, in the nucleic acid included in the endogenous gene of (i), an amino acid corresponding to position 163 of SEQ ID NO: 2 encoded by the nucleic acid is glycine. The amino acid residue relevant to the "amino acid corresponding to position 163 of SEQ ID NO: 2" can be recognized by comparison with SEQ ID NO: 2, as in "corresponding to the amino acids at positions from 233 to 285 of SEQ ID NO: 2". WO 2014/096283 describes an EMS mutant including a missense mutation in which position 163 of the S genome (SEQ ID NO: 2) of NtCLCa is mutated from glycine to arginine. In one embodiment, in a nucleic acid included in the endogenous gene of (i), the amino acid corresponding to position 163 of SEQ ID NO: 2 encoded by the nucleic acid is not arginine. In one embodiment, in a nucleic acid included in the endogenous gene of (i), the codon corresponding to the amino acid at position 163 of SEQ ID NO: 2 encoded by the nucleic acid does not include a mutation.

In one embodiment, the tobacco plant further includes:
(iii) an endogenous gene including, as a coding region, a nucleic acid including a nucleotide sequence encoding an amino acid sequence of SEQ ID NO: 8 or having at least 95% identity with SEQ ID NO: 8 in which an amino acid corresponding to position 231 of the amino acid sequence is proline.

SEQ ID NO: 6 is a tobacco homolog of AtCLCe which is a CLC family of *Arabidopsis,* and is the amino acid sequence of a protein encoded by an NtCLCe-S gene located on the S genome of *Nicotiana tabacum* (Nt). SEQ ID NO: 5 is the nucleotide sequence of CDS of the NtCLCe-S gene. The tobacco plant may or may not include a mutation in the NtCLCe-S gene.

SEQ ID NO: 8 is a tobacco homolog of AtCLCe which is a CLC family of *Arabidopsis,* and is the amino acid sequence of a protein encoded by an NtCLCe-T gene located on the T genome of *Nicotiana tabacum* (Nt). SEQ ID NO: 7 is the nucleotide sequence of CDS of the NtCLCe-T gene. The amino acid residue relevant to the "amino acid corresponding to position 231 of SEQ ID NO: 8" can be recognized by comparison with SEQ ID NO: 8. WO 2014/096283 describes an EMS mutant including a missense mutation in which position 143 of the NtCLCe protein (SEQ ID NO: 13 in the publication) derived from the NtCLCe-T gene is mutated from proline to leucine. Here, position 143 of SEQ ID NO: 13 in the publication corresponds to position 231 of SEQ ID NO: 8 herein. In one embodiment, in a nucleic acid included in the endogenous gene of (iii), the amino acid corresponding to position 231 of SEQ ID NO: 8 encoded by the nucleic acid is not leucine. In one embodiment, in a nucleic acid included in the endogenous gene of (iii), the codon corresponding to the amino acid at position 231 of SEQ ID NO: 8 encoded by the nucleic acid does not include a mutation.

In one embodiment, the tobacco plant is a mutant or may be a gene-modified variant.

Herein, the "mutant" is a modified variant of a tobacco plant by a random mutation caused naturally or artificially. The method for producing the mutant will be described in detail in "3. Method for producing tobacco plant" below. The mutant is not limited, but may be a tobacco plant obtained by selecting a tobacco plant including one or both of endogenous genes below from progenies obtained by crossbreeding of a tobacco plant with a mutant as one parent, or may be a progeny of the selected tobacco plant:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

The "gene-modified variant" is a tobacco plant in which an endogenous gene is modified by:
(i) introducing a mutation into an endogenous gene including, as a coding region, a nucleic acid including a nucleotide sequence encoding an amino acid sequence of SEQ ID NO: 2 or having at least 95% identity with SEQ ID NO: 2 such that at least one of codons corresponding to the amino acid sequence is mutated to a stop codon; and/or
(ii) introducing a mutation into an endogenous gene including, as a coding region, a nucleic acid including a nucleotide sequence encoding an amino acid sequence of SEQ ID NO: 4 or having at least 95% identity with SEQ ID NO: 4 such that at least one of codons corresponding to the amino acid sequence is mutated to a stop codon, and a progeny thereof is included.

In one embodiment, the tobacco plant of the present invention includes one or both of:
an endogenous gene including, as a coding region, a nucleotide sequence encoding a polypeptide lacking an amino acid sequence from an amino acid residue between positions 233 to 285 to the C-terminal amino acid residue in an amino acid sequence of SEQ ID NO: 2 or having at least 95% identity with SEQ ID NO: 2; and
an endogenous gene including, as a coding region, a nucleotide sequence encoding a polypeptide lacking an amino acid sequence from an amino acid residue between positions 233 to 285 to the C-terminal amino acid residue in an amino acid sequence of SEQ ID NO: 4 or having at least 95% identity with SEQ ID NO: 4; and a progeny thereof is included in the tobacco plant of the present invention.

Here, the polypeptide lacking an amino acid sequence from an amino acid residue between positions 233 to 285 to the C-terminal amino acid residue in the amino acid sequence of SEQ ID NO: 2 or 4 (including amino acid sequences having at least 95% identity therewith) may be a polypeptide not including an amino acid sequence from an amino acid residue between positions 233 to 285 to the C-terminal amino acid residue in the amino acid sequence of SEQ ID NO: 2 or 4. That is, the polypeptide can include "a polypeptide not including the amino acid sequence from an amino acid residue between positions 233 to 285 to the C-terminal amino acid residue in the amino acid sequence of SEQ ID NO: 2 or 4 but including an amino acid sequence, that is other than the amino acid sequence of SEQ ID NO: 2 or 4, generated by a frameshift as the C-terminal region".

The polypeptide lacking an amino acid sequence from an amino acid residue between positions 233 to 285 to the C-terminal amino acid residue in the amino acid sequence of SEQ ID NO: 2 or 4 (including amino acid sequences having at least 95% identity therewith) may be an embodiment in which at least one of codons corresponding to the amino acids at positions 233 to 285 in the amino acid sequence of SEQ ID NO: 2 or 4 is mutated to a stop codon and/or an embodiment in which a frameshift is caused by deletion or insertion of a nucleotide in the nucleic acid sequence from 697th to 855th of the CDS sequence of SEQ ID NO: 1 or 3.

The tobacco plant includes, for example, a mutant such as an EMS mutant and a gene-modified variant by genome editing or the like. In one embodiment, the tobacco plant is a mutant or a gene-modified variant.

The "mutant" and "gene-modified variant" can include not only a mature tobacco plant and the whole thereof but also portions thereof. The portions are unlimitedly selected from the group consisting of leaves (including leaf blades and petioles), stems, roots, seeds, flowers, pollens, anthers, ovules, pedicels, meristematic tissues, cotyledons, hypocotyls, pericycles, embryos, albumens, explants, calluses, tissue cultures, sprouts, cells, and protoplast.

In one embodiment, the tobacco plant of the present invention further includes:
(iv) a nitrate reductase in which an amino acid residue corresponding to position 525 in the amino acid sequence corresponding to SEQ ID NO: 42 or 44 is mutated from proline to an amino acid residue other than proline. The amino acid residue other than proline is preferably leucine or serine.

*Nicotiana tabacum* has two highly homologous nitrogen metabolism enzyme genes referred to as NIA1 (derived from the T genome) and NIA2 (derived from the S genome). The nitrate reductase of (iv) includes NIA1 protein encoded by the NIA1 gene and a mutant thereof and/or NIA2 protein encoded by the NIA2 gene and a mutant thereon.

The NIA1 gene and the NIA2 gene have nucleotide sequences of SEQ ID NOs: 41 and 43, respectively. The NIA1 protein and the NIA2 protein have the amino acid sequences of SEQ ID NOs: 42 and 44 encoded by the nucleotide sequences of SEQ ID NOs: 41 and 43, respectively (provided that the amino acid residue corresponding to position 525 is mutated from proline to an amino acid residue other than proline). The amino acid sequence of the mutated nitrate reductase of (iv) may be a mutant having slight variety (mutation) in SEQ ID NO: 2 or 4, as long as the condition that the amino acid residue corresponding to position 525 in the amino acid sequence corresponding to SEQ ID NO: 2 or 4 is mutated from proline to an amino acid residue other than proline is satisfied.

The present inventors found that in a tobacco plant having a nitrate reductase in which the amino acid residue corresponding to position 525 in the amino acid sequence of the NIA1 protein or the NIA2 protein is mutated from proline to an amino acid residue other than proline, the nitrate amount in leaves decreases and also the growth of the plant body is good (PCT/JP 2021/45295, WO 2022/124361).

The nitrate amount in leaves largely decreases, compared to a control, in a tobacco plant including (i) an endogenous gene (NtCLCa-S gene) including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon and/or (ii) an endogenous gene (NtCLCa-T gene) including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon and having a nitrate reductase in which the amino acid residue corresponding to position 525 in the amino acid sequence of the NIA1 protein or the NIA2 protein is mutated from proline to an amino acid residue other than proline (Example 5). Herein, the NtCLCa-S gene and the NtCLCa-T gene may be respectively an endogenous gene including, as a coding region, a nucleotide sequence encoding a polypeptide lacking an amino acid sequence from an amino acid residue between positions 233 to 285 to the C-terminal amino acid residue in an amino acid sequence of SEQ ID NO: 2 or having at least 95% identity with SEQ ID NO: 2 and an endogenous gene including, as a coding region, a nucleotide sequence encoding a polypeptide lacking an amino acid sequence from an amino acid residue between positions 233 to 285 to the C-terminal amino acid residue in an amino acid sequence of SEQ ID NO: 4 or having at least 95% identity with SEQ ID NO: 4.

### 2. Properties of tobacco plant

The tobacco plant of the present invention has one or more properties of the following (a) to (c):
(a) a nitrate content decreased compared to a control,
(b) a nitrite content equivalent to a control; and
(c) an amino acid content increased compared to a control.

Here, the control is a tobacco plant including a polypeptide containing the amino acid sequence of SEQ ID NO: 2 and a polypeptide containing the amino acid sequence of SEQ ID NO: 4.

The "tobacco plant including a polypeptide containing the amino acid sequence of SEQ ID NO: 2 and a polypeptide containing an amino acid sequence of SEQ ID NO: 4" as a control is a tobacco plant not including a stop codon in codons corresponding to the amino acid sequence and including the full-length NtCLCa-S protein (SEQ ID NO: 2) and NtCLCa-T protein (SEQ ID NO: 4). The tobacco plant as a control may be *Nicotiana tabacum,* preferably, wild type *Nicotiana tabacum.* The wild type *Nicotiana tabacum* includes the full-length NtCLCa-S protein (SEQ ID NO: 2) and NtCLCa-T protein (SEQ ID NO: 4), and neither the expression nor activity thereof has been modified in any way.

In one embodiment, the tobacco plant of the present invention has two or more properties or all of three properties of the above (a) to (c).

### (a) Nitrate content decreased compared to control

In one embodiment, the nitrate amount contained in the tobacco plant decreases compared to a control. Unlimitedly, nitrate can be measured by, for example, the method described in "(3) Nitrate analysis" in Example 3 herein. For example, leaves collected from a tobacco plant are cured and extracted with water, and the filtered filtrate may be then used as a measurement sample.

In one embodiment, nitrate in the tobacco plant is preferably decreased by at least 50%, at least 55%, at least 60%, at least 65%, at least 70%, at least 75%, at least 80%, at least 85%, at least 90%, at least 92%, or at least 95% compared to the control. In one embodiment, nitrate in the tobacco plant is decreased by at least 80% compared to a control.

### (b) Nitrite content equivalent to control

In one embodiment, nitrite contained in the tobacco plant is (substantially) equivalent to a control. Nitrite can be measured by, for example, the method described in "(4) Nitrite analysis" in Example 3 herein. For example, a powder prepared by curing leaves collected from a tobacco plant is extracted with water, and nitrite may be quantitatively measured by a colorimetric method using the filtered filtrate as a measurement sample.

"The nitrite content is equivalent to a control" means unlimitedly that the increase and decrease of nitrite is 30% or less, 28% or less, 25% or less, 23% or less, 20% or less, 18% or less, or 15% compared to the control. In one embodiment, "the nitrite content is equivalent to a control" means unlimitedly that the increase of nitrite is 30% or less, 28% or less, 25% or less, 23% or less, 20% or less, 18% or less, or 15% or less compared to a control.

The tobacco plant preferably has characteristics that although the nitrate content decreases, the nitrite content is equivalent to control.

### (c) Amino acid content increased compared to control

In one embodiment, the amino acids contained in the tobacco plant increases compared to a control. In one embodiment, the amino acids are free amino acids. (Free) amino acids can be measured by, for example, the method described in "(5) Free amino acid analysis" in Example 3 herein. For example, a solution containing a powder prepared by curing leaves collected from a tobacco plant is ultrasonicated, and the amino acids may be measured by HPLC analysis

In one embodiment, the amino acids contained in the tobacco plant increase by at least 1.1 times, 1.3 times, 1.4 times, 1.5 times, 1.8 times, or 2 times compared to the control. There is no particular upper limit of the incremental amount of amino acids. In one embodiment, the incremental amount of amino acids contained in the tobacco plant is 4 times or less, 3.5 times or less, 3 times or less, 2.8 times or less, 2.5 times or less, or 2.2 times or less compared to the control. In one embodiment, the amino acids contained in the tobacco plant increase by 1.1 to 4 times, 1.3 to 3.5 times, 1.4 to 3 times, or 1.5 to 2.5 times compared to the control. In one embodiment, the amino acids contained in the tobacco plant increase by about 2 times compared to the control.

The tobacco plant of the present invention may further have the following property (d).

### (d) Growth of individual is essentially equivalent to control

In one embodiment, the tobacco plant has a nitrate reductase consisting of the amino acid sequence of SEQ ID NO: 2 or 4, and the growth of an individual is essentially equivalent to a tobacco plant control.

"The growth of an individual is essentially equivalent to a control" means that the sizes (growing) such as the height of the plant body, the number of leaves, and the sizes of leaves, the mass (for example, the dry matter weight of leaves above the ground (biomass)), the anthesis, and so on of individuals are substantially the same. It is unlimitedly meant that, for example, when the heights are compared, the difference therebetween is 20% or less, 15% or less, 10% or less, or 5% or less.

For example, as shown in Example 4 herein, the tobacco plant had a nitrate reductase consisting of the amino acid sequence of SEQ ID NO: 2 or 4, and the lamina weight was equivalent to a tobacco plant control. In Example 5, also when pyramiding of mutant alleles of NtNIA1 (accumulation of genetic mutations) was performed, no influence on the tobacco growth was observed

One or more properties of the above (a) to (d) are preferably inherited not only in the M1 generation, which is a mutant or gene-modified variant, but also in subsequent generations (M2 generation, M3 generation, and beyond).

### 3. Method for producing tobacco plant

In one embodiment, the present invention relates to a method for producing a tobacco plant.

The method for producing a tobacco plant includes:
(i) introducing a mutation into a tobacco plant such that in an endogenous gene including, as a coding region, a nucleic acid including a nucleotide sequence encoding an amino acid sequence of SEQ ID NO: 2 or having at least 95% identity with SEQ ID NO: 2, at least one of codons corresponding to the amino acid sequence is mutated to a stop codon; and/or
(ii) introducing a mutation into the tobacco plant such that in an endogenous gene including, as a coding region, a nucleic acid including a nucleotide sequence encoding an amino acid sequence of SEQ ID NO: 4 or having at least 95% identity with SEQ ID NO: 4, at least one of codons corresponding to the amino acid sequence is mutated to a stop codon.

Alternatively, the method for producing a tobacco plant includes selecting a tobacco plant including one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

Introduction of a mutation into an endogenous gene (modification of endogenous gene) may be performed using, for example, a genome editing system. The genome editing system can introduce a nonsense mutation due to deletion, insertion, or substitution of a nucleotide at an arbitrary position in an endogenous gene including, as a coding region, a nucleic acid including a nucleotide sequence encoding, "an amino acid sequence of SEQ ID NO: 2 or having at least 95% identity with SEQ ID NO: 2" or "an amino acid sequence of SEQ ID NO: 4 or having at least 95% identity with SEQ ID NO: 4" such that at least one of codons corresponding to the amino acid sequence is mutated to a stop codon. The endogenous gene can be modified by a genome editing system including a site-specific nuclease that cleaves the CDS (SEQ ID NO: 1) of a gene of the S genome of NtCLCa and/or the CDS (SEQ ID NO: 3) of a gene of the T genome of NtCLCa. The genome editing system can also use nuclease-mediated non-homologous end joining or homologous recombination repair. The site-specific nuclease that is used in the genome editing system can be appropriately modified. For example, a modified site-specific nuclease may be a CRISPR/Cas9 system, ZFN, or TALEN. The site-specific nuclease can cleave an NtCLC gene. As the genome editing system, a modified CRISPR/Cas system such as a modified CRISPR/Cas-9 system, a modified transcriptional activator-like effector nuclease, a modified Zinc finger nuclease, or a modified meganuclease may be used.

The method for producing a tobacco plant may include selecting a tobacco plant including one or both of the following endogenous genes by, for example, a mutation:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

The method for causing a mutation in a plant is well-known in the art, and a mutation can be caused in a CLC gene of a plant using a mutagen that mainly causes a point mutation and short deletion, insertion, nucleotide substitution, and/or transfer including a chemical mutagen or radiation. Examples of the chemical mutagen include, but not limited to, ethyl methanesulfonate (EMS), methyl methanesulfonate, N-ethyl-N-nitrosourea, triethylmelamine, N-methyl-N-nitrosourea, procarbazine, chlorambucil, cyclophosphamide, diethyl sulfate, an acrylamide monomer, melphalan, nitrogen mustard, vincristine, dimethylnitrosamine, N-methyl-N'-nitro-nitrosoguanidine, nitrosoguanidine, 2-aminopurine, 7,12-dimethyl-benz(a)anthracene, ethylene oxide, hexamethylphosphoramide, bisulfane, diepoxyalkanes (diepoxyoctane, diepoxybutane, and those similar thereto), 2-methoxy-6-chloro-9[3-(ethyl-2-chloroethyl)aminopropylamino]acridine dihydrochloride, and formaldehyde. Examples of the radiation include, but not limited to, γ-rays, heavy ion beams, X-rays, neutron rays, and UV.

The process of selecting a tobacco plant in which a desired position in the NtCLCa gene is mutated to a stop codon by a mutation may include one or more crossbreeding steps. In one embodiment, a seed after mutagenesis may be sown and cultivated to obtain a first-generation plant body, and second-generation plant bodies obtained by self-pollination of the first-generation plant body may be screened for mutants. An advantage of screening of the second-generation plant bodies is that the mutation is derived from reproductive cells. The object to be mutagenized is not limited, but may be a seed or pollen. When pollen is mutagenized, plant bodies raised from seeds obtained by crossbreeding the pollen with a plant body that is not mutagenized may be screened for mutants.

Selection of a gene-modified variant or mutant including a stop codon at an objective position can be performed by, but not limited to, for example, extracting the genomic DNA from a tobacco plant, amplifying the DNA by PCR or the like, and analyzing the nucleotide sequence of the DNA encoding an amino acid sequence corresponding to SEQ ID NO: 2 or 4. Besides, the selection is possible by, for example, a method for detecting a difference between sequences by a difference between electrophoresis distances using an SSCP (single strand conformation polymorphism) method or a method for detecting the presence or absence of a mutation by cleaving a mismatch site using T7 Endonuclease I or the like.

In one embodiment, the present invention may be a selection nucleic acid marker to be used for selecting a tobacco plant including an endogenous gene including, as a coding region, a nucleic acid including a nonsense mutation and/or a frameshift mutation that causes a mutation to an objective stop codon in a nucleic acid including a nucleotide sequence encoding "an amino acid sequence of SEQ ID NO: 2 or having at least 95% identity with SEQ ID NO: 2" or "an amino acid sequence of SEQ ID NO: 4 or having at least 95% identity with SEQ ID NO: 4". Alternatively, the present invention may be a detection polynucleotide to be used for detecting a mutation to an objective stop codon. In one embodiment, the selection nucleic acid marker or the detection polynucleotide may be a nucleic acid amplification primer for amplifying a nucleotide sequence of a DNA encoding a portion containing the amino acid residues at positions from 233 to 285 of SEQ ID NO: 2 or the amino acid residues at positions from 233 to 285 of SEQ ID NO: 4, a primer for sequencing a nucleotide sequence of a DNA encoding a portion containing the amino acid residues at positions from 233 to 285 of SEQ ID NO: 2 or the amino acid residues at positions from 233 to 285 of SEQ ID NO: 4, or a probe that binds to a DNA encoding a portion containing the amino acid residues at positions from 233 to 285 of SEQ ID NO: 2 or the amino acid residues at positions from 233 to 285 of SEQ ID NO: 4. A person skilled in the art can appropriately select these selection nucleic acid marker or detection polynucleotide based on well-known techniques.

In one embodiment, the present invention may be a tobacco plant obtained by crossbreeding a tobacco plant having a mutation to an objective stop codon with a tobacco plant having a decreased nitrate content by a mechanism different from that of the tobacco plant of the present invention, and a progeny thereof.

### 4. Leaf tobacco and cured leaves

In one embodiment, the present invention relates to leaf tobacco harvested from the tobacco plant of the present invention. The present invention also relates to cured leaves generated from leaf tobacco of the present invention.

The meaning of the "tobacco leaves of the present invention" is as described in "3. Method for producing tobacco plant" and the part previous thereto.

The process of producing cured leaves from leaf tobacco is not particularly limited, and a well-known method can be used. The leaves of the tobacco plant are harvested and can be used as a material for producing a tobacco product and so on. The tobacco leaves may be air-cured, fire-cured, flue-cured, or sun-cured. Unlimitedly, in the air curing, tobacco is hung in a well-ventilated storeroom and exposed to air for 4 to 8 weeks for curing. The fire-cured tobacco is obtained by hanging tobacco in a large storeroom and continuously or intermittently heat-curing the leaves with fire for from 3 days to 10 weeks depending on the process and the tobacco. The flue-cured tobacco is obtained by hanging tobacco in a row in a curing barn and raising the temperature slowly, usually, over about 1 week for curing. The sun-cured tobacco is obtained by curing in the sun. This method is used for producing oriental leaf tobacco in Turkey, Greece, and other Mediterranean countries.

In one embodiment, the present invention relates to cured leaves derived from leaf tobacco of the present invention.

### 5. Cut filler, powder, sheet, stem, granule, extract, and composition

In one embodiment, the present invention relates to a cut filler, powder, sheet, stem, granule, or extract produced from cured leaves of the present invention.

The meaning of the "cured leaves of the present invention" is as described in "4. Leaf tobacco and cured leaves" and the part previous thereto.

The "cut filler" is cured tobacco leaves shredded into a long and narrow strip shape and is used for cigarettes.

The "stem" is the thickest leaf vein portion running at the center of a leaf.

The "powder" is cured tobacco leaves ground into a powdery shape.

The "granule" is the powder formed into a granular shape.

The "extract" is that obtained by extracting a material such as leaves or stems ("portions" preferably including "non-proliferative portions") derived from a tobacco plant for the purpose of improving the flavor of a tobacco product or the purpose of decreasing the content of a specific component in a tobacco product. As the extracting method, a well-known method for extracting essential oil, a specific component, or the like from a plant can be used.

Unlimitedly, the present invention relates to a composition containing a tobacco plant (including portions) or cured leaves of the present invention or a tobacco material (such as a cut filler) derived therefrom. The composition may use the tobacco plant or its portion directly or may use them by cutting, grinding, or milling into a small piece, slurry, or fine powder shape. As the composition, the tobacco plant or its portion harvested from farmland or the like may be used directly, may be used after dissipating part of moisture indoors or outdoors for a predetermined period of time, or may be used after dissipating most of moisture with a dryer or the like.

Unlimitedly, the composition may contain a cut filler, powder, sheet, stem, granule, or extract produced from cured leaves of the present invention.

In one embodiment, the cut filler, powder, sheet, stem, granule, extract, and composition of the present invention contain non-proliferative portions of the tobacco plant.

In one embodiment, the cut filler, powder, sheet, stem, granule, extract, and composition of the present invention include a nucleic acid including a nucleotide sequence encoding an amino acid sequence corresponding to SEQ ID NO: 2 and/or SEQ ID NO: 4 in which at least one of codons corresponding to the amino acid sequence is mutated to a stop codon.

### 6. Product

In one embodiment, the present invention relates to a tobacco product containing the cured leaves of the present invention and/or the cut filler, powder, sheet, stem, granule, or extract of the present invention.

The meaning of "the cured leaves of the present invention and/or the cut filler, powder, sheet, stem, granule, or extract of the present invention" is as described in "5. Cut filler, powder, sheet, stem, granule, extract, and composition" and the part previous thereto.

The type of the "tobacco product" is not particularly limited, and examples thereof include cigars, pipe tobacco, snuff tobacco (including snus and snuff), chewing tobacco, cut tobacco (including fine-cut), and hookahs, in addition to cigarettes. Furthermore, a non-combustion heating-type tobacco product that uses aerosol generated by heating tobacco as an aerosol source, a non-heating-type tobacco product for inhaling the flavor of tobacco without heating it, and so on are included

Unlimitedly, the "tobacco product" includes non-proliferative portions of a tobacco plant.

In one embodiment, the present invention provides the use of the tobacco plant, leaf tobacco, cured leaves, and composition of the present invention for producing a tobacco product.

In one embodiment, the present invention relates to the tobacco plant, leaf tobacco, cured leaves, and composition of the present invention to be used for producing a tobacco product.

In one embodiment, the present invention includes a method for producing a tobacco product. Unlimitedly, the method for producing a tobacco product includes preparing a tobacco plant-derived material from a tobacco plant including one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.
The production method may include a process of collecting leaves from the tobacco plant and preparing cured leaves. The method for producing a tobacco product can use a well-known method. For example, a tobacco product can be produced by curing leaves (leaf tobacco) collected from a tobacco plant of the present invention and performing a raw material process (rating, vein removal, adjustment and drying, and storage and aging), a raw material processing process (sheeting, extraction, granulating, heating, and flavoring), and a product process (blending, shredding, rolling, and packing).

The definition of the "tobacco product" is as described above.

### EXAMPLES

The present invention will now be described in detail based on Examples but is not limited to these Examples A person skilled in the art can easily add modification and change to the present invention based on the description of the present specification, and such modification and change are within the technical scope of the present invention.

### Example 1: Isolation of NtCLCa mutant

In this Example, CLCa mutants of *Nicotiana tabacum* were isolated:
a CDS sequence (SEQ ID NO: 1) of a wild type NtCLCa-S gene located on the S genome; and
a CDS sequence (SEQ ID NO: 3) of a wild type NtCLCa-T gene located on the T genome.

First, seeds of tobacco (variety: Tsukuba 1) were treated with EMS to produce a mutant library Mutants having a nonsense mutation in the NtCLCa gene were selected from this mutant library by sequencing the genome. The NtCLCa gene is a tobacco homolog of the AtCLCa gene. One mutant of the NtCLCa-S gene located on the S genome and two mutants of the NtCLCa-T gene located on the T genome were isolated. The selection was performed by the following method

### (1) Preparation of genomic DNA

A leaf piece approximately 5 mm square was placed in a 96-well deep well plate or a 2-mL tube, 200 to 500 µL of a DNA extraction buffer (0.2 M Tris-HCl, pH 8.0, 0.4 M NaCl, 25 mM EDTA, 0.5% SDS) and beads (96-well deep well plate) or one metal cone (2-mL exclusive tube) were added thereto, followed by milling with ShakeMaster NEO (BioMedical Science) at 1000 rpm for 3 to 5 minutes. Centrifugation was performed at 4400 rpm (96-well deep well plate) or 12000 rpm (2 mL exclusive tube) for 20 minutes, and the supernatant was collected. Nucleic acids were precipitated from the supernatant by an ethanol precipitation method and were suspended in 50 to 100 µL of a TE buffer.

### (2) PCR

PCR was performed using the genomic DNA prepared as described above as a template with KOD One (TOYOBO Co., Ltd.) or TKS Gflex DNA Polymerase (Takara Bio Inc.). The following reaction conditions were applied to the PCR reaction composition according to the attached manual.

KOD One: 94°C for 2 minutes, (98°C for 10 seconds, 60°C for 15 seconds, and 68°C for 5 seconds) × 40 cycles, and 68°C for 5 minutes,

TKS Gflex DNA Polymerase: 94°C for 1 minute, (98°C for 10 seconds, 55°C for 15 seconds, and 68°C for 1 minute) × 40 cycles, and 68°C for 1 minute.

Primers were appropriately produced based on the sequence information of each gene and were used for selection. The primers used for isolation and analysis of a nonsense mutation were as follows wherein 220S is a primer for S gene analysis, and 222T and 223T are primers for T gene analysis:
220S-F: ATTACCGGCTCAGGTGGCGT (SEQ ID NO: 9), 220S-R: TGCCAGATTTGCAGTATTCA (SEQ ID NO: 10)
222T-F: CTTACCAACTCCTTTTTCCC (SEQ ID NO: 11), 222T-R: TGTGATAAAAAGACCATGT (SEQ ID NO: 12)
223T-F: ATAATTACCGGCTCAAATGG (SEQ ID NO: 13), 223T-R: TGTGATAAAAAGACCATGT (SEQ ID NO: 14)

### (3) Sequence analysis

Prior to sequencing reaction of a PCR product, the PCR product was purified using ExoSAP-IT (registered trademark) For PCR Product Clean-UP (Affymetrix, Inc.) referring to the attached protocol. The sequencing reaction was carried out using BigDye Terminator v.3.1 cycle sequencing kit (Thermo Fisher Scientific Inc.) by a reaction of the method attached to the kit (94°C for 30 seconds, 96°C for 10 seconds/50°C for 5 seconds/60°C for 2 minutes × 25 cycles). Subsequently, DNA was purified using BigDye Xterminator (trademark) Purification Kit (Thermo Fisher Scientific Inc.) by the method attached to the kit (45 µL of SAM Solution and 10 µL of XTerminater were added to each sample, followed by shaking for 30 minutes and then centrifugation at 1000g for 2 minutes). Sequence information was obtained with Applied Biosystems (registered trademark) 3730 DNA Analyzer (Thermo Fisher Scientific Inc.) and analyzed with sequence assembly software ATGC (GENETYX Corporation).

As a result of screening, one mutant including a stop codon in the NtCLCa-S gene was isolated. It is a mutant (220S) in which the codon (TGG) encoding the 279th tryptophan (W) was mutated to a stop codon (TAG). Two mutants each including a stop codon in the NtCLCa-T gene were isolated. They are a mutant (222T) in which the codon (TGG) encoding the 233rd tryptophan (W) was mutated to a stop codon (TAG) and a mutant (223T) in which the codon (TGG) encoding the 285th tryptophan (W) was mutated to a stop codon (TAG). Hereinafter, the NtCLCa-S mutant gene including the mutant 220S may be referred to as an NtCLCa-S-Δ1 gene, the NtCLCa-T mutant gene including the mutant 222T may be referred to as an NtCLCa-T-Δ1 gene, and the NtCLCa-T mutant gene including the mutant 223T may be referred to as an NtCLCa-T-Δ2 gene.

For each of the NtCLCa-S gene and the NtCLCa-T gene, M2 individuals homozygous for the mutant gene were bred to generate an F1 strain, and the F1 strain was further self-pollinated to obtain an F2 strain. For the F2 strain, the nucleotide sequence of each gene was analyzed by the above-described methods (1) to (3). Consequently, it was determined whether each individual was a homozygote or heterozygote of the mutant gene or a wild type without the mutation.

The nitrate and nitrite in leaves were analyzed using the isolated strains of the F2 generation The results obtained for independent two strains of the F2 generation were that in the homozygote of the mutant gene in which both the NtCLCa-S gene and the NtCLCa-T gene had a nonsense mutation, the nitrate concentration in leaves was significantly decreased compared to wild type individuals without the mutation (the method was as described in Example 3, no data are shown).

### Example 2: Selection of F3 strain

In this Example, F3 strains were selected

Self-pollinated F3 strains were obtained using the isolated strains of F2 generation obtained in Example 1. As Line 1, self-pollinated F3 seeds of the respective separated two individuals of the F2 generation: HeHo (1-7) that is a heterozygote of the wild type NtCLCa-S gene and the NtCLCa-S-Δ1 gene and a homozygote of the NtCLCa-T-Δ2 gene; and WHe (1-55) that is a homozygote of the wild type NtCLCa-S gene and a heterozygote of the wild type NtCLCa-T gene and the NtCLCa-T-Δ2 gene, were obtained.

As Line 2, self-pollinated F3 seeds of the respective separated individuals of the F2 generation: HoHe (2-9) that is a homozygote of the NtCLCa-S-Δ1 gene and a heterozygote of the wild type NtCLCa-T gene and the NtCLCa-T-Δ1 gene and WW (2-54) that is a homozygote of the wild type NtCLCa-S gene and a homozygote of the wild type NtCLCa-T, were obtained.

The following 3 types of plants were selected from self-pollinated F3 strains of the individual (1-7): HoHo that is homozygous for both the NtCLCa-S-Δ1 gene and the NtCLCa-T-Δ2 gene; HeHo that is a heterozygote of the wild type NtCLCa-S gene and the NtCLCa-S-Δ1 gene and a homozygote of the NtCLCa-T-Δ2 gene; and WHo that is a homozygote of the wild type NtCLCa-S gene and a homozygote of the NtCLCa-T-Δ2 gene. WW that is a homozygote of the wild type NtCLCa-S gene and a homozygote of the wild type NtCLCa-T was selected from self-pollinated F3 strains of the individual (1-55).

In addition, from the self-pollinated F3 strains of the individual (2-9), the following 3 types of plants were selected: HoHo that is homozygous for both the NtCLCa-S-Δ1 gene and the NtCLCa-T-Δ2 gene; HoHe that is a homozygote of the NtCLCa-S-Δ1 gene and a heterozygote of the wild type NtCLCa-T gene and the NtCLCa-T-Δ1 gene; and HoW that is an individual homozygous for the NtCLCa-S-Δ1 gene and homozygous for the wild type NtCLCa-T. WW that is an individual including a homozygote of the wild type NtCLCa-S gene and a homozygote of the wild type NtCLCa-T was obtained from self-pollinated F3 strains of 2-54.

### Example 3: Analysis of F3 strain

In this Example, the F3 strains obtained in Example 2 were analyzed

### (1) Cultivation

Cultivation from sowing seeds to sampling was performed with an artificial meteorological device Koitotron (KGBH-2416, Koito Electric Industries, Ltd.). The cultivation conditions from sowing to transplantation were a 16-hour daylength, a room temperature of 28°C, and a humidity of 60%.

The seeds of the F3 strains obtained in Example 2 were sown, and temporary planting was performed after about 2 weeks, followed by further cultivation for about 2 weeks. Subsequently, the young plants were transplanted in 12-cm terracotta filled with 500 to 600 mL of vermiculite. After the transplantation, the young plants were cultivated for about 3 weeks under the cultivation conditions of a light period at 25°C and a dark period at 18°C, a 12-hour daylength, and a humidity of 60% (light period)/80% (dark period). After the transplantation, about 100 mL of a nitrate liquid fertilizer (20 mM as NO₃: 4 mM Ca(NO₃)₂, 4 mM Mg(NO₃)₂, 4 mM KNO₃) was given per day to each individual.

### (2) Preparation of analysis sample

Twenty days after the transplantation, the lamina of 6 leaves above the ground of each individual was sampled, packed in a paper bag, and dried at a constant temperature of 80°C using a forced circulation thermostat (Isuzu Seisakusho). The dried lamina was ground into a dry powder. This dry powder was used for analysis of nitrate, nitrite, and free amino acids.

### (3) Nitrate analysis

One milliliter of Milli-Q water was mixed per 0.1 g of the dry powder prepared in "(2) Preparation of analysis sample", followed by shaking extraction at 240 pm at room temperature for 1 hour or more. The extract was filtered, and the filtrate was measured for the nitrate concentration using Ntrachek 404 Meter (KPG Products Ltd.) in accordance with the attached manual. The liquid volume to be dropped to an MQuant test strip (MIlliporeSigma) was set to 7 to 8 µL, and the average of values measured twice for each sample was used as the measurement value. The measurement was performed by diluting the sample such that the measurement value would be 100 ppm or less.

The results are shown in Fig. 1. The measured nitrate concentration was converted to nitrate nitrogen. The nitrate concentration in leaves of HoHo was 1/20 or less of that in WW as a control not having a mutation in the NtCLCa gene. In also HoHe, HeHo, HoW, and WHo, it was observed a significant decrease in the nitrate concentration in leaves compared to WW as a control not having a mutation in both genes.

### (4) Nitrite analysis

Nitrite was quantitatively determined using the filtrate prepared in the "(3) Nitrate analysis" by a colorimetric method. A hundred microliters of the filtrate was mixed with 50 µL of a color reagent 1 (2% sulfanilamide in 3 N HCl), and the mixture was reacted at room temperature for 5 minutes; 50 µL of a color reagent 2 (0.1% N-naphthyl ethylenediamine in water) was added thereto and mixed therewith; and the mixture was reacted at room temperature for 10 minutes. A540 and A700 as a reference were measured with a microplate reader Infinite 200 PRO (TECAN), and A540 - A700 (difference) was defined as a measured value. The quantitative measurement was performed using NaNOz (FUJIFILM Wako Pure Chemical Corporation, special grade) as a standard.

The results are shown in Fig. 2. No significant increase or decrease in the nitrite concentration in leaves was observed depending on the presence or absence of the nonsense mutation in the NtCLCa gene. Even in HoHo that showed a dramatic decrease in nitrate, no increase in the nitrite concentration was observed This point was an unexpected result that differed from the analysis results of the CLCa mutant of *Arabidopsis thaliana* (NPL 2).

### (5) Free amino acid analysis

Twenty milliliters of an 80% methanol solution (V/V) was added to and mixed with 0.4 g of the dry powder prepared in "(2) Preparation of analysis sample", followed by ultrasonication for 30 minutes. The sonicated solution was centrifugated (3000 rpm × 5 minutes). The supernatant after centrifugation was analyzed using an HPLC analyzer (Agilent 1290 infinity), an analysis column (Agilent ZORBAX Eclipse AAA, 3.5 µm, 3.0 × 150 mm), and a guard column (Agilent ZORBAX Eclipse AAA, 5 µm, 4.6 × 12.5 mm, 4/PK) under conditions of a mobile phase A: 40 mM phosphate buffer, a mobile phase B: 45% acetonitrile/45% methanol aqueous solution, and gradient: yes. Only HoHo and WW as a control were analyzed for 19 free amino acids.

The results are shown in Table 1 and Fig. 3.

**[Table 1]**

| Free amino acids | F3 lines | | | |
|---|---|---|---|---|
| | CLCa_1_HoHo | CLCa_1_WW | CLCa_2_HoHo | CLCa_2_WW |
| Alanine | 1918±452 | 1306±446 | 2093±473 | 1106±272 |
| Arginine | 350±115 | 205±45 | 483±194 | 188±28 |
| Asparagine | 2493±526 | 1405±253 | 2713±756 | 870±247 |
| Aspartic acid | 511±111 | 635±241 | 423±236 | 335±59 |
| Glutamine | 10589±2684 | 6010±2828 | 11378±4071 | 2748±1440 |
| Glutamic acid | 143±24 | 479±289 | 192±129 | 230±79 |
| Glycine | 790±176 | 289±98 | 975±463 | 350±103 |
| Histidine | 155±57 | 107±25 | 175±51 | 64±7 |
| Isoleucine | 240±68 | 196±25 | 250±113 | 133±27 |
| Leucine | 681±147 | 486±90 | 748±216 | 346±62 |
| Lysine | 381±142 | 272±44 | 494±112 | 149±29 |
| Methionine | 112±24 | 63±20 | 144±30 | 45±8 |
| Norvaline | 579±87 | 411±111 | 625±47 | 358±87 |
| Phenylalanine | 518±80 | 442±77 | 631±166 | 334±52 |
| Proline | 6395±952 | 5365±369 | 5500±844 | 3138±420 |
| Serine | 634±85 | 392±77 | 660±264 | 265±53 |
| Threonine | 820±161 | 516±107 | 753±300 | 349±46 |
| Tyrosine | 634±112 | 548±141 | 860±167 | 310±27 |
| Valine | 492±93 | 335±72 | 523±169 | 235±44 |

| | | | | |
|---|---|---|---|---|
| N = 5, average ± SD Unit is µg/g. | | | | |

The concentrations of 17 amino acids excluding at least glutamic acid and aspartic acid in leaves were higher in HoHo than in WW in both Line 1 and Line 2 (Table 1).

The total concentration of 19 free amino acids was 1.5 to 2.6 times higher in HoHo than in WW (Fig. 3). This point was also an unexpected result that differed from the analysis results of the CLCa mutant of *Arabidopsis thaliana* (NPL 1).

### (6) Growth

In order to compare growth, 20 days after transplantation, individuals of each genotype were placed side by side, and photographs thereof were taken (Fig. 4). No difference in the growth of F3 individuals was observed in both Line 1 and Line 2 regardless of the presence or absence of the nonsense mutation of the NtCLCa gene. These results demonstrated that the use of a mutant having a nonsense mutation in the NtCLCa gene can significantly decrease the nitrate concentration in leaves without affecting the growth and can increase the free amino acid concentration without increasing the nitrite concentration

### (7) Verification of sequence of NtCLCe gene

It was verified as follows that in the mutant of the NtCLCa gene obtained in Example 2, the CDS sequence of the NtCLCe gene did not have a mutation.

RNA was extracted from young leaves of individuals of 2 strains of F3 strain, HoHo of Line 1, and HoHo of Line 2 obtained in Example 2 using RNeasy Plant Mini Kit (QIAGEN N. V.). Complementary DNA was prepared using this RNA as a template using PrimeScript (trademark) RT reagent Kit with gDNA Eraser (Takara Bio Inc.). PCR primers were designed from estimated CDS sequence information of the respective NtCLCe genes of S genome and T genome (estimated from the nucleotide sequence information of the NtCLCe gene described in PTL 1), and PCR was performed using the cDNA as the template. The PCR was performed using TKS Gflex DNA Polymerase (Takara Bio Inc.) as the PCR enzyme in accordance with the attached manual under the following reaction conditions: 94°C for 1 minute, (98°C for 10 seconds, 55°C for 15 seconds, and 68°C for 1 to 2 minutes) × 40 cycles, and 68°C for 1 to 2 minutes.

The list of the used primer sequences is shown in Table 2 below (SEQ ID NOs: 15 to 27).

**[Table 2]**

| RT-PCR primer | | | |
|---|---|---|---|
| Name | Sequence (5'-3') | Use | Target genome |
| S/T_FW1 | TTGGTCTGACGAAGTTGTACG | PCR, sequence | S |
| S_RT_F1 | ATCGCTTCTGGATTTAATGC | PCR, sequence | S, T |
| S_RT_F2 | ATATGGCCTGGTTGGCATGG | PCR, sequence | S |
| S/T_RT_R1 | TTCATGAACCGCAGCGTTGA | sequence | S, T |
| S_RT_R2 | GCATTAAATCCAGAAGCGAT | PCR, sequence | S, T |
| S_RT_R3 | AGCCTTTCCTTCGTGGAGAA | PCR, sequence | S |
| S_RT_R4 | AACCGCAGTGAGAGGCACCT | sequence | S |
| T_RT_F1 | TCAACGCTGCGGTTCATGAA | sequence | S, T |
| T_RT_F2 | GTACACCTACTGAGCTGCCG | PCR, sequence | S, T |
| T_RT_F3 | TTATGGCCTGGTTGGCATG | PCR, sequence | S, T |
| T_RT_F4 | CTCATTGGTCTGCTGACACTT | sequence | S, T |
| T_RT_R2 | CGGCAGCTCAGTAGGTGTAC | PCR, sequence | S, T |
| T_RT_R3 | GAAGAAGAGGCTTGAACATCAG | PCR, sequence | T |

As a result of PCR using the following primer pairs, cDNA sequences of the NtCLCe genes of S and T genomes were amplified.
S/T_FW1 and S_RT_R2;
S_RT_F1 and S RT R3;
S_RT_F2 and S_RT_R3;
S/T_FW1 and S_RT_R3;
S/T_FW1 and T_RT_R2;
T_RT_2 and T_RT_R3;
T_RT_F3 and T_RT_R3; and
S/T FW1 and T_RT_R3.

The sequences of the PCR products were analyzed by the method described in "(3) Sequence analysis" of Example 1, and as a result, all sequences matched. Accordingly, it was confirmed that the NtCLCe gene sequence in the mutant of the NtCLCa gene obtained in Example 2 is exactly the same as the wild type of Tsukuba 1.
CDS sequence (SEQ ID NO: 5) of the NtCLCe-S gene located on the S genome; and
CDS sequence (SEQ ID NO: 7) of the NtCLCe-T gene located on the T genome.

### (8) Daily fluctuation of nitrate in leaves

HoHo individuals and WW individuals of the F3 strains of Line 2 obtained in Example 2 were cultivated as described in "(1) Cultivation". Twenty days after transplantation, leaves were sampled 1, 3, 5, and 10 and a half hours after the start of the light period and 4 and 8 hours after the start of the dark period. The timings of the sampling correspond to early morning, mid-morning, before noon, 1.5 hours before sunset, 4 hours after sunset, and 8 hours after sunset, respectively. Nitrate and nitrite in the lamina were quantitatively determined as described in "(3) Nitrate analysis" and "(4) Nitrite analysis". The results are shown in Figs. 5 and 6. The nitrate concentration in the lamina of HoHo was lower than WW regardless of day and night (Fig. 5). The nitrite concentration in the lamina was almost at the same level between HoHo and WW regardless of day and night (Fig. 6).

### (9) Analysis of expression of NtCLCa and NtCLCe genes in NtCLCa mutant

Unfolded leaves of 10 individuals of each of HoHo and WW of the F3 strain of Line 1 obtained in Example 2 were analyzed for the expression of each gene of NtCLCa and NtCLCe.

Ten leaf disks with a diameter of 5 mm were collected from each individual and mixed, and RNA was extracted using NucleoSpin Plant II (Takara Bio Inc.). The concentration of the extracted RNA was measured using Nanodrop 8000 (Thermo Fisher Scientific Inc.).

The reaction below was performed using 750 ng of RNA of each sample. Complementary DNA was prepared using each RNA as the template and using ReverTra Ace (registered trademark) qPCR RT Master Mix with gDNA Remover (TOYOBO Co., Ltd.). The cDNA synthesized from 10 ng of RNA, THUNDERBIRD (registered trademark) SYBR qPCR Mix (TOYOBO Co., Ltd.), and primers shown in Table 3 were mixed, and the expression level of each gene was measured using StepOne (trademark) (Applied Biosystems).

### (SEQ ID NOs: 28 to 37)

**[Table 3]**

| Primer set | Primer name | Sequence (5'-3') |
|---|---|---|
| CLCa set 1 | CLCa_qRT-PCR_F3 | CATGGACGGACAACAGAATG |
| | CLCa_qRT-PCR_R3 | GGGAGACCTTAGCTCCAACC |
| CLCa set 2 | CLCa_qRT-PCR_F7 | ATTGGTTCACATTGGCGCTT |
| | CLCa_qRT-PCR_R7 | ATAAGATCTCGCCTGTCCCG |
| CLCe set 1 | CLCa_qRT-PCR_F4 | CTTGCTTTGTTGGCCTCTTC |
| | CLCa_qRT-PCR_R4 | TGTACTCCAATGGGCTCCTC |
| CLCe set 2 | CLCa_qRT-PCR_F6 | ACGAAAGTTGTTGGGTCGTC |
| | CLCa_qRT-PCR_R6 | TATCGTCTTTGCTGCTGTCG |
| Control | ribosomal protein L25_qRT_F | CCCCTCACCACAGAGTCTGC |
| | ribosomal protein L25_qRT_R | AAGGGTGTTGTTGTCCTCAATCTT |

The results are shown as the average of expression levels obtained by relative quantification (Δ Δ Ct method) of an object using ribosomal protein L25 (Accession No. L18908) as the control gene (Mol. Genet. Genomics, (2010), 283: 233-241). The expression level of the NtCLCa gene of HoHo was significantly lower than WW, about half of WW, in both 2 pairs of primers used for the expression analysis (Fig. 7). In contrast, there was no significant difference in the expression level of the NtCLCe gene between HoHo and WW (Fig. 8).

PTL 1 discloses that in RNAi recombinant tobacco using the sequence of the CLC-Nt2-s gene (corresponding to NtCLCa-S), expression of the NtCLCe gene is also suppressed at the same time. That is, a decrease of nitrate that is observed in the RNAi recombinant tobacco using the sequence of the CLC-Nt2-s gene is a consequence of suppressing the expression of both the NtCLCa and NtCLCe genes. In contrast, it was revealed that the decrease of nitrate that is observed in the NtCLCa mutant of the present invention is not accompanied by suppression of expression of the NtCLCe gene.

### (10) Verification that 163rd amino acid of NtCLCa-S gene is glycine in NtCLCa mutant

NtCLCa-S gene fragments were specifically amplified by PCR using the cDNAs prepared from the NtCLCa mutants (HoHo) of Lines 1 and 2 obtained in Example 2 as the templates. The PCR was performed using the cDNA prepared in "(7) Verification of sequence of NtCLCe gene" and using TKS Gflex DNA Polymerase (Takara Bio Inc.) as the PCR enzyme in accordance with the attached manual under the following reaction conditions: 94°C for 1 minute, (98°C for 10 seconds, 55°C for 15 seconds, and 68°C for 1 minute) × 40 cycles, and 68°C for 12 minutes.

As the primers, the following 2 sets were used.
CLCA_S_F1 (5'-CATGACTGGAGAAGGAGATCT-3') and
CLCA_S_R1 (5'-AAGTGTGGTTGCTCCATACATA-3')
(SEQ ID NOs: 38 and 39)
CLCA_S_F1 (5'-CATGACTGGAGAAGGAGATCT-3') and
CLCa_220s_R (5'-TGCCAGATTTGCAGTATTCA-3')
(SEQ ID NOs: 38 and 40)

Sequences were analyzed using these PCR amplification products as templates by the method described in (3) Sequence analysis of Example 1. As a result, it was confirmed that the nucleic acid sequence corresponding to the 163rd amino acid of the NtCLCa-S gene was glycine.

### CDS sequence of NtCLCa-S gene (SEQ ID NO: 1)

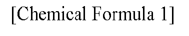

ATGGAGGAGCCAACTCGATTAGTAGAAGAAGCAACGATTAATAACATGGACGGACAACAGAATGAA
GAAGAAAGAGATCCAGAGAGCAATTCACTGCATCAGCCTCTTCTCAAGAGAAACAGAACACTATCA
TCCAGTCCATTTGCCTTGGTTGGAGCTAAGGTCTCCCACATCGAAAGTTTGGATTATGAGATCAAC
GAGAATGATCTCTTCAAGCATGACTGGAGAAGGAGATCTAGAGTTCAAGTATTACAGTATGTGTTC
TTGAAATGGACACTGGCATTTTTGGTCGGCCTGCTTACAGGAGTTACAGCCACCCTCATCAATCTT
GCAATCGAAAACATGGCTGGTTACAAACTTCGAGCTGTTGTGAACTATATCGAGGATAGAAGGTAC
CTTATGGGATTTGCATATTTTGCGGGTGCTAATTTTGTGCTCACTTTGATAGCTGCCCTTCTCTGCG
TGTGCTTTGCACCTACTGCTGCAGGGCCTGGAATTCCTGAAATCAAAGCTTATCTCAACGGTGTAG
ATACTCCCAATATGTATGGAGCAACCACACTTTTTGTCAAGATCATTGGAAGCATTGCAGCAGTTT
CTGCTAGCTTAGACCTTGGAAAAGAAGGGCCATTGGTTCACATTGGCGCTTGCTTTGCTTCCTTAC
TAGGTCAAGGTGGTCCAGATAATTACCGGCTCAGGTGGCGTTGGCTCCGTTACTTCAACAACGATC
GGGACAGGCGAGATCTTATCACATGTGGGTCATCATCAGGTGTGTGTGCTGCTTTCCGTTCTCCAG
TAGGTGGTGTCCTATTTGCTTTAGAGGAAGTGGCAACATGGTGGAGAAGTGCACTCCTCTGGAGAA
CTTTCTTCAGCACGGCAGTTGTGGTGGTGATACTGAGGGCCTTCATTGAATACTGCAAATCTGGCA
ACTGTGGACTTTTTGGAAGAGGAGGGCTTATCATGTTTGATGTGAGTGGTGTCAGTGTTAGCTACC
ATGTTGTGGACATCATCCCTGTTGTAGTGATTGGAATCATAGGCGGACTTTTGGGAAGCCTCTACA
ATCATGTCCTCCACAAAATTCTGAGGCTCTACAATCTGATCAACGAGAAGGGAAAACTACATAAGG
TTCTTCTCGCTCTGAGTGTCTCCCTTTTCACCTCCATTTGCATGTATGGACTTCCTTTTTTGGCCAA
ATGCAAGCCTTGTGATCCATCACTTCCCGGGTCTTGTCCTGGTACTGGAGGGACAGGAAACTTCAA
GCAGTTCAACTGCCCAGACGGCTATTACAATGATCTTGCTACTCTTCTCCTTACAACCAACGATGA
TGCAGTCCGAAACATTTTCTCCATAAACACTCCCGGTGAATTCCAAGTTATGTCTCTTATTATCTAC
TTCGTTCTGTATTGCATATTGGGACTCATCACTTTTGGGATTGCTGTGCCATCTGGTCTCTTCCTTC
CAATCATCCTCATGGGTTCAGCTTATGGTCGCTTGCTTGCCATTGCCATGGGATCTTATACAAAAA
TTGATCCAGGGCTGTATGCGGTTCTCGGAGCAGCTTCCCTTATGGCTGGTTCAATGAGAATGACTG
TTTCTCTTTGCGTCATATTTCTTGAGCTAACAAACAATCTTCTCCTTCTGCCAATAACAATGCTGGT
TCTTCTAATTGCCAAAAGTGTAGGAGACTGCTTCAACCTAAGTATTTATGAAATAATATTGGAGCTG
AAAGGTCTACCTTTCCTGGATGCCAACCCGGAGCCATGGATGAGAAATATCACTGCTGGTGAGCTT
GCTGATGTAAAGCCACCAGTAGTTACACTCTGTGGAGTTGAGAAGGTGGGACGTATCGTAGAGGC
CTTGAAGAACACCACATATAACGGATTCCCTGTCGTCGATGAAGGAGTAGTGCCACCGGTGGGTCT
GCCAGTTGGGGCAACTGAATTGCACGGTCTTGTCCTAAGAACTCACCTTCTTTTGGTTCTCAAGAA
AAAGTGGTTCCTTCATGAAAGACGGAGGACAGAGGAGTGGGAAGTGAGAGAGAAATTCACCTGGA
TTGATTTAGCTGAGAGGGGCGGTAAGATCGAAGATGTGTTAGTTACAAAGGATGAAATGGAGATGT
ATGTCGATTTGCATCCCCTGACTAACACAACCCCTTATACTGTGGTAGAAAGCTTGTCAGTGGCTA
AGGCAATGGTGCTTTTCAGGCAGGTGGGGCTCCGCCACATGCTCATTGTACCCAAATACCAAGCA
GCAGGGGTATCTCCTGTGGTGGGAATCTTGACCAGGCAAGACTTGAGAGCCCACAACATTTTGAGT
GTCTTCCCTCATCTGGAGAAGTCAAAAAGCGGTAAAAAGGGGAACTGA

The underlined sequence corresponds to the 163rd glycine.

### Example 4: Biomass amount of plant cultivated in the field

In this Example, the lamina weight of leaves of the F3 plant obtained in Example 2 was investigated

HoHo and WW derived from Lines 1 and 2 were cultivated according to a usual tobacco cultivation method. Fig. 9 shows plants 54 days after the transplantation (Line 1).

The plants were top pruned on the 70th day from the transplantation. The 9th and 10th leaves from the top of each of 12 individual plants were sampled 15 days after the top pruning, and the 5th and 6th leaves from the top of other 12 individual plants were sampled 18 days after the top pruning. The stem (midrib) was immediately removed from the sampled leaves, and the lamina was dried at 70°C for 2 days and at 50°C for 1 day. Table 4 shows the results of measurement of the weight of the dry lamina obtained from two leaves. The lamina weight of HoHo was equivalent to WW that is a control not having mutant alleles of NtCLCa-S and NtCLCa-T.

**[Table 4]**

| Line | Genotype | 9th and 10th leaves | | | 5th and 6th leaves | | |
|---|---|---|---|---|---|---|---|
| | | Mean | SD | p value | Mean | SD | p value |
| Line1 | HoHo | 16.826 | 2.109 | 0.2432 | 16.849 | 2.749 | 0.4123 |
| | WW | 15.560 | 2.987 | | 15.941 | 2.573 | |
| Line2 | HoHo | 15.863 | 1.962 | 0.2194 | 14.139 | 1.358 | 0.5628 |
| | WW | 14.838 | 2.009 | | 14.559 | 2.071 | |

### Example 5: Gene pyramiding

WO 2022/124361 (International publication of International Application PCT/JP2021/045295 by the same applicant as this application) discloses a tobacco plant including a nitrate reductase in which the amino acid residue corresponding to position 525 in the amino acid sequence (SEQ ID NO: 2) of NIA1 protein or the amino acid sequence (SEQ ID NO: 4) of NIA2 protein is mutated from proline to an amino acid residue (preferably, leucine or serine) other than proline. This literature discloses that in the tobacco plant, the nitrate amount in leaves decreases, and the growth of the plant body is also good.

In this Example, influence of pyramiding of the mutant allele of NtNIA1 (accumulation of genetic mutations) in a tobacco plant having mutant alleles of NtCLCa-S and NtCLCa-T on the nitrate accumulation amount of the tobacco plant was investigated.

As the tobacco plant having mutant alleles of NtCLCa-S and NtCLCa-T, HoHo in which the NtCLCa-S-Δ1 gene and the NtCLCa-T-Δ2 gene derived Line 1 obtained in Example 2 were both homozygous was used. In breeding for gene pyramiding, a homozygote (P525L_Homo) of the NtNIA1 mutant allele encoding P525L-NR protein was used. This homozygote is a BC2F3 plant obtained by crossbreeding of EMS mutant (M3) and a recurrent parent TN90 (Burley type) described in Example 7 of WO 2022/124361.

From the F2 group obtained by crossbreeding the both, an F2 plant holding a homozygous NtCLCa-S mutant allele, a heterozygous NtCLCa-T mutant allele, and a heterozygous NtNIA1 mutant allele was selected. The selected F2 plant is a double homozygote holding a recessive allele of two genes NtEGY1 and NtEGY2. This recessive allele is associated with a frameshift mutation and is thought to be a cause of chlorophyll deficiency and low nitrogen use efficiency in Burley-type tobacco (BMC genomics, (2017), 18(1), 1-14).

Furthermore, from an F3 group derived from this F2 plant, F3 plants showing 4 genotypes shown in Table 5 were selected and cultivated by the method described in Example 3.

**[Table 5]**

| Genotype | NtCLCa-S | NtCLCa-T | NtNIA1 |
|---|---|---|---|
| HoHoW | Ho | Ho | W |
| HoHoHo | Ho | Ho | Ho |
| HoHoHe | Ho | Ho | He |
| HoHeHo | Ho | He | Ho |

In Table 5, Ho, He, and W show the plants are a homozygote of a mutant allele, a heterozygote of a mutant allele and a wild type allele, and a homozygote of a wild type allele, respectively.

Leaves were sampled from the plants 20 days after the transplantation by the method described in Example 3 and were dried, and the nitrate nitrogen content was then analyzed (n = 6). The results are shown in Table 6 (p value is a value of a two-sided test between the genotype HoHoW and each genotype).

### [Table 6]

**Table 6: Nitrate nitrogen content (ppm)**

| Genotype | Mene. | SD | n | p value |
|---|---|---|---|---|
| HoHoW | 198.1 | 32.3 | 6 | |
| HoHoHo | 90.9 | 23.5 | 6 | 6.230E-05 |
| HoHoHe | 96.2 | 23.6 | 6 | 9.654E-05 |
| HoHeHo | 2021.0 | 361.8 | 6 | 2.330E-07 |

The HoHoHo plant and HoHoHe plant had a significantly low nitrate nitrogen compared to the HoHoW plant. Accordingly, pyramiding of the NtNIA1 mutant allele in the tobacco plant having mutant alleles of NtCLCa-S and NtCLCa-T further decreased the nitrate nitrogen content in the tobacco plant.

Fig. 10 shows a photograph of the plants 20 days after the transplantation. Pyramiding of the NtNIA1 mutant allele in the tobacco plant having mutant alleles of NtCLCa-S and NtCLCa-T did not affect the growth of the tobacco plant.

### INDUSTRIAL APPLICABILITY

The TSNA of a tobacco raw material or a tobacco product can be decreased by using the tobacco plant of the present invention. In the tobacco plant of the present invention, not only the nitrate amount in leaves decreases, but also preferably the growth of the plant body is good, and the amino acid content increases. The tobacco plant of the present invention can be used as a material of excellent leaf tobacco for producing a tobacco product.

## Claims

1. A tobacco plant comprising one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

2. The tobacco plant according to claim 1, fulfilling one or both of following requirements:
in the nucleic acid included in the endogenous gene of (i), at least one of codons corresponding to amino acids at positions from 233 to 285 of SEQ ID NO: 2 is mutated to a stop codon; and
in the nucleic acid included in the endogenous gene of (ii), at least one of codons corresponding to amino acids at positions from 233 to 285 of SEQ ID NO: 4 is mutated to a stop codon.

3. The tobacco plant according to claim 1 or 2, comprising both endogenous genes of (i) and (ii).

4. The tobacco plant according to any one of claims 1 to 3, wherein the endogenous gene of (i) and/or the endogenous gene of (ii) is a homozygote.

5. The tobacco plant according to any one of claims 1 to 4, wherein in the nucleic acid included in the endogenous gene of (i), a codon corresponding to an amino acid at position 279 of SEQ ID NO: 2 is mutated to a stop codon.

6. The tobacco plant according to any one of claims 1 to 5, wherein in the nucleic acid included in the endogenous gene of (ii), a codon corresponding to an amino acid at position 285 of SEQ ID NO: 4 is mutated to a stop codon.

7. The tobacco plant according to any one of claims 1 to 5, wherein in the nucleic acid included in the endogenous gene of (ii), a codon corresponding to an amino acid at position 233 of SEQ ID NO: 4 is mutated to a stop codon.

8. The tobacco plant according to any one of claims 1 to 6, wherein
in the nucleic acid included in the endogenous gene of (i), a codon corresponding to an amino acid at position 279 of SEQ ID NO: 2 is mutated to a stop codon, and
in the nucleic acid included in the endogenous gene of (ii), a codon corresponding to an amino acid at position 233 or position 285 of SEQ ID NO: 4 is mutated to a stop codon.

9. The tobacco plant according to any one of claims 1 to 8, wherein in the nucleic acid included in the endogenous gene of (i), an amino acid corresponding to position 163 of SEQ ID NO: 2 encoded by the nucleic acid is glycine.

10. The tobacco plant according to any one of claims 1 to 9, further comprising:
(iii) an endogenous gene including, as a coding region, a nucleic acid including a nucleotide sequence encoding an amino acid sequence of SEQ ID NO: 8 or having at least 95% identity with SEQ ID NO: 8 in which an amino acid corresponding to position 231 of the amino acid sequence is proline.

11. The tobacco plant according to any one of claims 1 to 10, having one or more properties of following (a) to (c):
(a) a nitrate content decreased compared to a control;
(b) a nitrite content equivalent to a control; and
(c) an amino acid content increased compared to a control,
wherein the control is a tobacco plant including a polypeptide containing the amino acid sequence of SEQ ID NO: 2 and a polypeptide containing the amino acid sequence of SEQ ID NO: 4.

12. The tobacco plant according to claim 11, wherein nitrate is decreased by at least 80% compared to the control.

13. The tobacco plant according to any one of claims 1 to 12, wherein the tobacco plant is a mutant or a gene-modified variant.

14. The tobacco plant according to any one of claims 1 to 13, wherein the tobacco plant is *Nicotiana tabacum.*

15. A tobacco plant comprising one or both of:
an endogenous gene including, as a coding region, a nucleotide sequence encoding a polypeptide lacking an amino acid sequence from an amino acid residue between positions 233 to 285 to a C-terminal amino acid residue in an amino acid sequence of SEQ ID NO: 2 or having at least 95% identity with SEQ ID NO: 2; and
an endogenous gene including, as a coding region, a nucleotide sequence encoding a polypeptide lacking an amino acid sequence from an amino acid residue between positions 233 to 285 to a C-terminal amino acid residue in an amino acid sequence of SEQ ID NO: 4 or having at least 95% identity with SEQ ID NO: 4.

16. A method for producing the tobacco plant according to any one of claims 1 to 14, comprising:
(i) introducing a mutation into a tobacco plant such that in an endogenous gene including, as a coding region, a nucleic acid including a nucleotide sequence encoding an amino acid sequence of SEQ ID NO: 2 or having at least 95% identity with SEQ ID NO: 2, at least one of codons corresponding to the amino acid sequence is mutated to a stop codon; and/or
(ii) introducing a mutation into the tobacco plant such that in an endogenous gene including, as a coding region, a nucleic acid including a nucleotide sequence encoding an amino acid sequence of SEQ ID NO: 4 or having at least 95% identity with SEQ ID NO: 4, at least one of codons corresponding to the amino acid sequence is mutated to a stop codon.

17. A method for producing the tobacco plant according to any one of claims 1 to 14, comprising:
selecting a tobacco plant including one or both of:
(i) an endogenous gene including, as a coding region, a nucleic acid including a nucleotide sequence encoding an amino acid sequence of SEQ ID NO: 2 or having at least 95% identity with SEQ ID NO: 2 in which at least one of codons corresponding to the amino acid sequence is mutated to a stop codon; and
(ii) an endogenous gene including, as a coding region, a nucleic acid including a nucleotide sequence encoding an amino acid sequence of SEQ ID NO: 4 or having at least 95% identity with SEQ ID NO: 4 in which at least one of codons corresponding to the amino acid sequence is mutated to a stop codon.

18. The method according to claim 16 or 17, further comprising:
selecting a tobacco plant having a decreased nitrate content.

19. Leaf tobacco harvested from the tobacco plant according to any one of claims 1 to 14.

20. A cured leaf generated from the leaf tobacco according to claim 19.

21. A cut filler, powder, sheet, stem, granule, or extract produced from the cured leaf according to claim 20.

22. A tobacco product comprising the cured leaf according to claim 20 and/or the cut filler, powder, sheet, stem, granule, or extract according to claim 21.
